(19) Europäisches Patentamt
European Patent Office
Office européen des brevets

(11) **EP 2 486 956 B1**

(12) **EUROPÄISCHE PATENTSCHRIFT**

(45) Veröffentlichungstag und Bekanntmachung des Hinweises auf die Patenterteilung:
**03.06.2015 Patentblatt 2015/23**

(51) Int Cl.:
***A61N 5/10*** *(2006.01)*

(21) Anmeldenummer: **11154210.6**

(22) Anmeldetag: **11.02.2011**

(54) **Verfahren zur Identifikation möglicher Veränderungen der Reichweite eines geplanten Bestrahlungsfeldes vor der Bestrahlung eines Patienten mit geladenen Teilchen**

Method for the identification of possible changes in range of a planned radiation field before irradiating a patient with the loaded particles

Procédé d'identification de modifications éventuelles du rayon d'action d'un champ de rayonnement planifié avant l'irradiation d'un patient par des particules chargées

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**15.08.2012 Patentblatt 2012/33**

(73) Patentinhaber: **Rinecker, Hans**
**81379 München (DE)**

(72) Erfinder: **Rinecker, Hans**
**81379 München (DE)**

(74) Vertreter: **Wächter, Jochen et al**
**Kroher-Strobel**
**Rechts- und Patentanwälte PartmbB**
**Bavariaring 20**
**80336 München (DE)**

(56) Entgegenhaltungen:
**EP-A1- 1 380 262     EP-A1- 1 911 493**
**EP-A1- 2 229 981     US-A1- 2007 164 230**

EP 2 486 956 B1

**Beschreibung**

Die vorliegende Erfindung betrifft ein Verfahren zur Identifikation möglicher Veränderungen der Reichweite eines geplanten Bestrahlungsfeldes vor der Bestrahlung eines Patienten mit geladenen Teilchen.

Anlagen zur Bestrahlung von Patienten mit geladenen Teilchen besitzen im Vergleich zu konventionellen Bestrahlungsanlagen, die mit Röntgen- bzw. Photonenstrahlen arbeiten, erhebliche Vorteile hinsichtlich der Zielgenauigkeit der Dosisapplikation sowie der Verringerung der Nebenwirkungen im gesunden Gewebe.

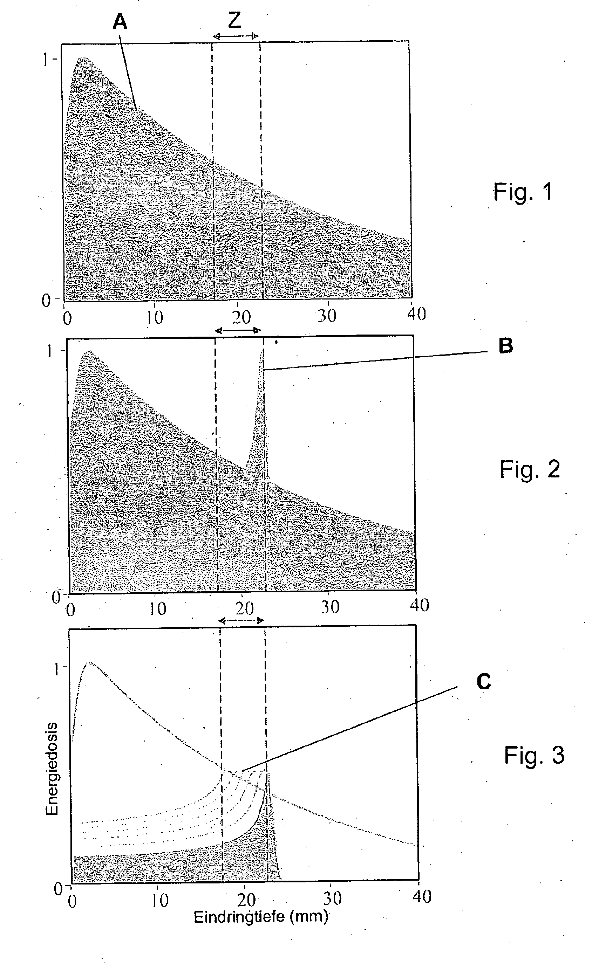

Konventionelle Photonenstrahlen durchdringen den Körper, werden aber auch bei ihrer Interaktion mit den Molekülen des Körpers absorbiert und verlieren dadurch stetig an Intensität. Das Dosismaximum liegt dabei knapp unter der Haut, wie aus der Dosisverteilungskurve A aus Fig. 1 ersichtlich ist. Dieser Effekt beruht auf der erst unmittelbar unter der Haut eintretenden Rekrutierung von Streustrahlung. Auf dem weiteren Weg in Richtung des Tumorbereichs Z fällt die Strahlendosis dann in Form einer Exponentialkurve ab. Ein tief im Körper liegender Tumor erhält somit weniger Dosis als gesundes Gewebe im Strahlengang vor ihm, während hinter dem Tumor liegende Organe immer noch eine beträchtliche Dosis erhalten.

Im Gegensatz hierzu geben geladene Teilchen wie Protonen und Schwerionen nach dem Eindringen in den Körper zunächst nur relativ wenig Energie ab, werden aber durch wiederholte Wechselwirkung mit Materie gebremst (siehe Fig. 2). Je langsamer die Partikel werden, desto höher sind die abgegebene Energie und die Abbremsung. Dies führt zu einer "Energieexplosion" am Ende der Teilchenbahn, dem sog. "Bragg-Peak" (Dosisverteilungskurve B in Fig. 2). Vor dem Tumor ist im Gegensatz zur Bestrahlung mit Photonen bei geladenen Teilchen die Dosis wesentlich niedriger bei gleichzeitiger Konzentration der Dosis in den Tumor. Da der Bragg-Peak aufgrund der definierten begrenzten Reichweite der Protonenstrahlen einen sehr scharfen Dosisabfall aufweist, bleibt bei Protonenbestrahlung der Patient hinter dem Tumor sogar strahlungsfrei. Die Beeinflussung der erzeugten Partikelgeschwindigkeit in Verbindung mit einer seitlichen Ablenkung erlaubt es also, die Dosis dreidimensional in den Tumor zu zielen. Dabei wird durch Variation der Teilchengeschwindigkeit auch in der Tiefe über den Tumor hinweg verfahren, wie aus Fig. 3 ersichtlich ist, in der ein Bragg-Plateau C dargestellt ist.

Ein bevorzugtes Verfahren, bei dem der Bragg-Peak des Strahls geladener Teilchen den Tumor millimetergenau in einem zuvor durch Diagnostik und Bestrahlungsplanung festgelegten dreidimensionalen Raster computergesteuert abfährt, ist das sog. Raster-Scan-Verfahren. Bei diesem Verfahren beträgt der Strahldurchmesser typischerweise 7-10 mm FWHM, und die einzelnen Rasterpunkte (typischerweise einige hundert bis einige tausend) werden nacheinander angesteuert und in jeder Bestrahlungssitzung in typischerweise 60 bis 90 Sekunden mit der jeweils festgelegten Dosis bestrahlt. Eine Patientenbehandlung erfolgt typischerweise in mehreren Bestrahlungssitzungen an aufeinander folgenden Tagen.

Insbesondere bei nahe an lebenswichtigen gesunden Strukturen liegenden Tumoren und sehr tief liegenden Tumoren, die man aufgrund der zwangsläufigen unerwünschten Schädigung des gesunden Gewebes in vielen Fällen überhaupt nicht, in anderen Fällen nur mit hohem Risiko mit herkömmlicher Photonenstrahlentherapie behandeln kann, stellt die Bestrahlung mit geladenen Teilchen einen erheblichen Fortschritt in der Krebsbehandlung dar.

Beim Bestrahlen mit geladenen Teilchen nach dem Raster-Scan-Verfahren bringt die oben erläuterte scharfe und konzentrierte Dosisverteilung des Bleistift-Strahls und die damit verbundene zielgenaue dreidimensionale Bestrahlung aber auch erhöhte Anforderungen an die einzuhaltende Genauigkeit mit sich.

Zur Erläuterung soll am Beispiel der Protonentherapie noch etwas weiter ausgeholt werden. Die Reichweite eines Protonenstrahls hängt nicht nur von der initialen Energie beim Eintritt in den Körper ab, sondern auch von der Beschaffenheit des Gewebes, welches auf dem Weg zum gewünschten Ort der Dosisdeposition im Körper (Tumor) vom Protonenstrahl durchlaufen wird.

Bei der Anwendung von Protonentherapie wird deshalb im initialen Schritt ein sogenannter Therapieplan erstellt. Hierbei werden zunächst möglichst hoch aufgelöste Computertomographiedaten (CT-Daten) für die Körperbereiche, in denen ein Bestrahlungszielgebiet liegt, erzeugt. Die darin enthaltenen Grauwerte (sog. Hounsfield-Einheiten) werden dann durch geeignete Kalibrierung des CT-Scanners übertragen in Elektronendichtewerte und letztlich in Bremsvermögen für Protonen überführt. In diese so erzeugten Schnittbilder (ggf. unter Hinzuziehung weiterer diagnostischer Modalitäten, wie z.B. Kernspintomographie) werden vom behandelnden Strahlentherapeuten die Konturen der Zielgebiete sowie die Konturen von zu schonenden kritischen Strukturen und Organen eingezeichnet. Hierdurch ergeben sich mittels Interpolation zwischen den Schnittbildern dreidimensionale Formen. In einem nächsten Schritt wird dann in der Regel durch den Medizinphysikexperten mit Hilfe von Computersystemen der Therapieplan erzeugt, der das Zielgebiet (Tumor) mit der gewünschten Dosis belegt und gleichzeitig das gesunde Gewebe schont.

Bei der Protonentherapie im Scanningverfahren werden dabei die Einstrahlrichtungen für die Protonen festgelegt und eine dreidimensionale Fluenzmatrix erzeugt, in der die Orte, Energien und Dosisgewichte der Einzelstrahlen (Scanning-Spots) enthalten sind. Nach der Optimierung dieser Fluenzmatrix erhält man die gewünschte Dosisverteilung (in der Regel homogen) im Zielgebiet unter optimaler Schonung der kritischen Strukturen. Das Therapieplanungssystem verwendet hierzu sämtliche Informationen aus dem dreidimensionalen CT-Datensatz sowie die eingezeichneten Konturen. Dies bedeutet, dass für jeden Einzelstrahl mit einem gegebenen Zielort (lateral x und y sowie Eindringtiefe z) auch sämtliche Dichtevariationen wie beispielsweise Muskel-, Knochen- oder Lungengewebe vollumfänglich hinsichtlich der Abbremsung des Protonenstrahls berücksichtigt sind.

Der so erstellte Therapieplan kann nun auf den Patienten angewendet werden. Unumgängliche Voraussetzung für eine erfolgreiche Behandlung ist dabei zunächst die Einhaltung der korrekten Position des Patienten während jeder Bestrahlung. Hierzu existiert eine Vielzahl von Lösungsansätzen, z.B. Vakuummatratzen, Beißblöcke, Immobilisationsmasken sowie Röntgensystem-gestützte Positionsverifikationssysteme.

Außerdem überprüfen Sicherheitseinrichtungen die Therapieplanungsdaten und die Funktionalität der Anlage, damit eine falsche Umsetzung der Therapieplanungsdaten und Daten-übertragungsfehler zwischen den einzelnen Komponenten der Anlage minimiert werden können.

Trotz all dieser Vorkehrungen können auch weitere Faktoren die Präzision der Bestrahlung und damit den Behandlungserfolg beeinflussen. Beispielsweise können Unterschiede in der Dichtezusammensetzung oder in der Verteilung der Dichtezusammensetzungen innerhalb des Patienten auftreten. Dies sind beispielsweise Gaseinschlüsse im Darm oder etwa Fettfalten (Lagerungsunterschiede an der Oberfläche des Patienten). Hierdurch können sich für eine gegebene Position eines Patienten und einen gegebenen Therapieplan, welcher auf der bei der CT-Aufnahme vorliegenden Dichteverteilung basiert, Reichweitenvariationen bei Einzelstrahlen des Therapieplans ergeben. Diese Reichweitenvariationen können unter Umständen auch bedeutsam sein für den Therapieerfolg, indem das Zielvolumen etwa nicht homogen mit Dosis versorgt wird oder indem ein Gebiet mit kritischer Struktur mit ungewollter Dosis belegt wird. Einer unbeabsichtigten Variation der Reichweite von Protonen-Einzelstrahlen kommt wegen des steilen Dosisabfalls in Strahlrichtung hinter dem Bragg-Peak eine wesentlich höhere klinische Bedeutung zu als bei einer Variation der Patientengeometrie bei der herkömmlichen Strahlentherapie mit Photonenstrahlen, die einen flachen exponentiellen Dosisabfall aufweist.

Die meisten dieser Einflüsse werden im Rahmen des Therapieplanungsoptimierungsprozesses berücksichtigt und fließen in die Maximierung der sogenannten Robustheit des Therapieplans ein. Da eine Strahlentherapie aber wie erwähnt in der Regel über mehrere Therapiesitzungen (Fraktionen) verteilt wird, kann es im tagesindividuellen Fall zu Dichte- und damit Reichweitenvariationen kommen, welche individuell zu überprüfen sind.

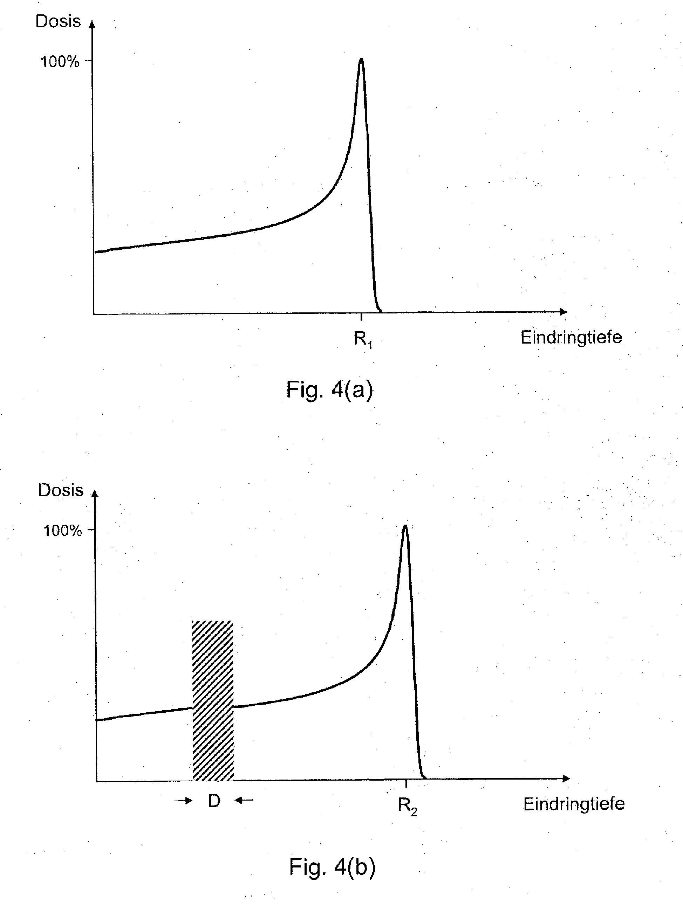

Fig. 4(a) und 4(b) dienen zur Veranschaulichung dieses Problems. Fig. 4(a) zeigt dabei noch einmal eine charakteristische Tiefendosiskurve eines Protonenstrahls in einem homogenen Medium. Die Kurve endet mit dem bekannten Bragg-Peak bei der Reichweite $R_1$.

Allgemein gilt, dass jedes hochenergetische Proton beim Eindringen in Materie eine von seiner initialen Energie und der Dichte sowie Zusammensetzungsverteilung des durchstrahlten Materials abhängige Reichweite hat. Diese unterliegt aufgrund der statistischen Natur der Wechselwirkungen der Protonen mit der Materie und somit des Energieverlustprozesses einer Schwankung. Für einen Protonenstrahl mit einer bestimmten initialen Energie wird die Tiefendosiskurve durch Integration der Energieverluste erzielt. Das Bremsvermögen dE/dx der Protonen in Materie ist hinreichend aus der Literatur bekannt und wird gut vom sogenannten Bethe-Bloch-Formalismus beschrieben.

Verändert sich die Dichtefunktion in einem Teilabschnitt des Einstrahlbereichs des Protonenstrahls, beispielsweise durch eine Luftblase (schraffierter Bereich D in Fig. 4(b)), welche vorherigen Darminhalt ersetzt, so ergibt sich eine Veränderung der Reichweite, im vorliegenden Beispiel eine Vergrößerung der Reichweite des Protonenstrahls hin zum Wert $R_2$. Grundsätzlich könnte, um dieser Problematik Herr zu werden, an jedem einzelnen Fraktionstag eine vollumfängliche Computertomographie sowie eine komplette Therapieplanung mit der tagesaktuellen Dichteverteilung durchgeführt werden. Dies scheidet jedoch aus diversen Gründen aus. Die Dosisbelastungen für die Patienten durch eine tägliche Computertomographie sind schon nicht vertretbar. Außerdem liegt der Zeitbedarf für eine vollständige Computertomographie mit Therapieplanung selbst bei optimaler Computer-Performance im Bereich von Stunden und ist deshalb nicht geeignet, da der Patient sich in der Zwischenzeit wiederum in seiner Dichteverteilung verändern könnte. Schließlich würde bei typischerweise getrennter räumlicher Anordnung der notwendige Patiententransport zwischen CT-Raum und Bestrahlungsraum wiederum zu Unsicherheiten in der Dichteverteilung und damit der Reichweite führen.

Der vorliegenden Erfindung liegt daher die Aufgabe zugrunde, ein Verfahren zur Identifikation möglicher Veränderungen der Reichweite eines geplanten Bestrahlungsfeldes vor der Bestrahlung eines Patienten mit geladenen Teilchen zu schaffen, das dem klinischen Anwender unmittelbar vor jeder Bestrahlungssitzung am bereits im Behandlungsraum positionierten Patienten schnell eine transparente Überprüfung der Bestrahlungsfertigkeit des Patienten ermöglicht und mit dem das Risiko von tagesindividuellen klinisch relevanten Reichweitenverschiebungen von Teilbereichen des Behandlungsfeldes im Patienten minimiert werden kann.

Erfindungsgemäß umfasst das Verfahren zur Identifikation möglicher Veränderungen der Reichweite eines geplanten Bestrahlungsfeldes vor der Bestrahlung eines Patienten mit geladenen Teilchen folgende Schritte:

- Durchführen einer Computertomographie über zumindest einen Teil des Patienten zur Erzeugung eines dreidimensionalen Computertomographiedatensatzes;
- Bereitstellen eines Röntgensystems aus zwei hochauflösenden Röntgendetektoren, die miteinander einen Winkel von 60° bis 120°, bevorzugt von 75° bis 105°, einschließen, und zwei den Röntgendetektoren gegenüberliegenden Röntgenquellen, wobei jedes Paar von Röntgenquelle und Röntgendetektor jeweils eine Projektionsrichtung definiert;

- Erstellen von zwei Ist-Röntgenbildern zumindest eines Teils des Patienten in den zwei Projektionsrichtungen mit den zwei Röntgendetektoren;
- Berechnen von zwei digitalen rekonstruierten Soll-Röntgenbildern aus dem Computertomographiedatensatz entsprechend den zwei Projektionsrichtungen des Röntgensystems;
- Durchführen einer Differenzanalyse zur Feststellung von Unterschieden zwischen Ist-Röntgenbild und zugehörigem Soll-Röntgenbild für die zwei Projektionsrichtungen;
- Überprüfen der Relevanz der festgestellten Unterschiede hinsichtlich räumlicher Ausdehnung und hinsichtlich Lokalisation in Bezug auf das geplante Bestrahlungsfeld und Festlegen von relevanten zweidimensionalen Variationsgebieten;
- Festlegen eines dreidimensionalen Variationskörpers durch Rückprojektion der beiden zweidimensionalen Variationsgebiete in den dreidimensionalen Computertomographiedatensatz;
- Berechnen einer mittleren Elektronendichte des dreidimensionalen Variationskörpers auf Basis der festgestellten Unterschiede zwischen Ist-Röntgenbild und zugehörigem Soll-Röntgenbild für die zwei Projektionsrichtungen;
- Identifizieren von Einzelstrahlen des geplanten Bestrahlungsfeldes, die durch den dreidimensionalen Variationskörper treten;
- Berechnen von zu erwartenden geänderten Reichweiten der identifizierten Einzelstrahlen des geplanten Bestrahlungsfeldes unter Berücksichtigung des geänderten Elektronendichtewerts im dreidimensionalen Variationskörper; und
- Darstellen oder Weiterleiten von Informationen über die berechneten geänderten Reichweiten.

Mit diesem Verfahren können auch kurzfristig auftretende Dichteschwankungen im Bestrahlungsweg auf ihre klinische Relevanz hin untersucht werden, und somit wird eine weitere Sicherheitsstufe für die punktgenaue Bestrahlung des Patienten eingeführt.

Vorzugsweise umfasst der Schritt des Durchführens einer Differenzanalyse zur Feststellung von Unterschieden zwischen Ist-Röntgenbild und zugehörigem Soll-Röntgenbild den Schritt, numerische Grauwerte von sich entsprechenden Pixeln des Ist-Röntgenbildes und Soll-Röntgenbildes zu subtrahieren, um eine pixelweise Grauwertabweichung zu bestimmen. Auf diese Weise wird die Auswertung der Unterschiede auf einem hohen Detaillierungsgrad möglich.

Vor dem Schritt des pixelweisen Subtrahierens der numerischen Grauwerte wird vorzugsweise eine Grauwertanpassung zwischen den Ist-Röntgenbildern und den Soll-Röntgenbildern durchgeführt, so dass eine eventuell vorhandene systembedingte Grauwertverschiebung zwischen Ist-Röntgenbildern und Soll-Röntgenbildern, die auf Unterschiede zwischen den Aufnahmemodalitäten der Ist-Röntgenbilder und der Berechnung der Soll-Röntgenbilder aus dem CT-Datensatz zurückzuführen ist, vor der Differenzbildung eliminiert wird.

Vorzugsweise umfasst das Durchführen der Grauwertanpassung zwischen Ist-Röntgenbild und Soll-Röntgenbild den Schritt, in einem iterativen numerischen Optimierungsverfahren eine Anpassungsfunktion zum Abgleich von Grauwerthäufigkeitskurven von Ist-Röntgenbild und Soll-Röntgenbild anzuwenden. Auf diese Weise können systembedingte Unterschiede zwischen Ist-Röntgenbildern und Soll-Röntgenbildern besonders robust und effizient ausgeblendet werden.

Zur Erhöhung der Detaillierung der gewonnenen Daten wird vorzugsweise vor dem Durchführen der Grauwertanpassung zwischen Ist-Röntgenbild und Soll-Röntgenbild die Auflösung desjenigen mit der geringeren Auflösung auf die höhere Auflösung des anderen angepasst.

Vorzugsweise werden beim Festlegen der relevanten zweidimensionalen Variationsgebiete ausschließlich Gebiete berücksichtigt, die aus einer vorbestimmten Mindestzahl zusammenhängender Pixel bestehen, die nach dem Subtrahieren eine Grauwertabweichung oberhalb eines vorbestimmten Schwellwerts zeigen. Auf diese Weise bleiben Fehlinformationen einzelner Pixel und Rauschartefakte für das weitere Verfahren unberücksichtigt.

Zur Vereinfachung der weiteren Rechenschritte wird vorzugsweise über die Pixel jedes relevanten zweidimensionalen Variationsgebiets eine Grauwertmittelung durchgeführt.

Um dem klinischen Nutzer sofort eine erste Einschätzung der Situation zu erlauben, wird vorzugsweise jedes relevante zweidimensionale Variationsgebiet in den Ist-Röntgenbildern oder den Soll-Röntgenbildern graphisch hervorgehoben.

Vor dem Rückprojizieren der relevanten zweidimensionalen Variationsgebiete in den dreidimensionalen Computertomographiedatensatz werden die zweidimensionalen Variationsgebiete vorzugsweise zu Rechtecken mit der mittleren Breite und mit der mittleren Länge des jeweiligen zweidimensionalen Variationsgebietes vereinfacht. Diese Maßnahme erlaubt eine weitaus schnellere rechnerische Rückprojektion in den dreidimensionalen Computertomographiedatensatz.

Vorzugsweise werden beim Berechnen der mittleren Elektronendichte des dreidimensionalen Variationskörpers Gleichungssysteme für jede der zwei Projektionsrichtungen basierend auf dem Röntgenabschwächungszusammenhang

$$G(r) = e^{-\alpha \cdot \rho \cdot r}$$

numerisch gelöst, wobei G der numerische Grauwert und p die Elektronendichte ist. Durch diese Methode gelingt es auf einfachste Weise, eine Näherung für die mittlere Elektronendichte im Variationskörper zu erhalten.

Das Berechnen von zu erwartenden geänderten Reichweiten der identifizierten Einzelstrahlen des geplanten Bestrahlungsfeldes weist vorzugsweise die Schritte auf, den differentiellen Energieverlust entlang des Eindringpfades des Einzelstrahls zu bestimmen und die Reichweite des Eindringpfades des Einzelstrahls durch Integration über den energieabhängigen differentiellen Energieverlust zu bestimmen. Damit können die geänderten Reichweiten effektiv näherungsweise berechnet werden.

Zur Beschleunigung des Rechenvorgangs trägt auch bei, wenn das Berechnen von zu erwartenden geänderten Reichweiten der identifizierten Einzelstrahlen des geplanten Bestrahlungsfeldes nur auf diejenigen Einzelstrahlen beschränkt wird, welche die höchsten ursprünglichen Reichweiten betreffen. Die klinische Relevanz des Verfahrens bleibt dabei unangetastet, weil die Einzelstrahlen mit den höchsten ursprünglichen Reichweiten die höchste Energieabgabe liefern und zudem die hintere Grenze des Bestrahlungsfeldes bilden.

Das Darstellen von Informationen über die berechneten geänderten Reichweiten umfasst vorzugsweise den Schritt, die Informationen visuell in den Ist-Röntgenbildern oder Soll-Röntgenbildern als Angaben über die Ausdehnung der Reichweitenveränderung darzustellen. So erhält der klinische Nutzer eine schnelle und aussagekräftige Information über die klinische Relevanz der Dichtevariation.

In einer komplexeren Stufe des Systems kann das Darstellen von Informationen über die berechneten geänderten Reichweiten den Schritt umfassen, in den Ist-Röntgenbildern oder Soll-Röntgenbildern eine Reichweitenvariation im distalen Endbereich des Bestrahlungsfeldes farblich zu kennzeichnen. Dadurch wird die Relevanz der Dichteabweichung visuell noch besser zugänglich.

Zur weiteren Verdeutlichung der klinischen Relevanz der Dichteabweichung kann schließlich in einer noch komplexeren Stufe des Systems das Darstellen von Informationen über die berechneten geänderten Reichweiten den Schritt umfassen, eine geänderte Dosisverteilung zu berechnen, die aus den berechneten geänderten Reichweiten folgt, und in den beiden Ist-Röntgenbildern oder Soll-Röntgenbildern Projektionen von Isodosisflächen der geänderten Dosisverteilung darzustellen.

Weitere Merkmale und Vorteile der vorliegenden Erfindung ergeben sich aus der nachfolgenden Beschreibung unter Bezugnahme auf die Zeichnungen:

Fig. 1            zeigt eine Dosisverteilungskurve bei der konventionellen Bestrahlung mit Photonen;

Fig. 2            zeigt eine Dosisverteilungskurve bei der Bestrahlung mit Protonen einer bestimmten Energie im Vergleich zur Dosisverteilungskurve bei der Bestrahlung mit Photonen;

Fig. 3            zeigt die Überlagerung verschiedener Dosisverteilungskurven bei der Bestrahlung mit Protonen zur Abtastung des Tumors;

Fig. 4 (a) und 4(b)    zeigen den Effekt einer Reichweitenänderung eines Protonenstrahls in unterschiedlich dichter Materie;

Fig. 5            zeigt den schematischen Aufbau einer Anlage zur Bestrahlung von Patienten mit geladenen Teilchen;

Fig. 6            zeigt eine Detaildarstellung eines Ausführungsbeispiels des 3D-Scanning-Systems;

Fig. 7            zeigt eine schematische Übersicht über die elektronischen Komponenten einer erfindungsgemäßen Anlage, welche an der Berechnung und Übertragung von Bestrahlungsdaten und Bestrahlungsbefehlen beteiligt sind;

Fig. 8            ist eine schematische Querschnittsansicht der Anordnung des Röntgensystems im Behandlungsraum;

Fig. 9(a) bis 9(c)    zeigen schematisch den Ablauf der Differenzanalyse zur Feststellung von Unterschieden zwischen Ist-Röntgenbild und zugehörigem Soll-Röntgenbild;

Fig. 10(a) bis 10(c)      zeigen schematisch die Grundzüge einer Grauwertanpassung zwischen Ist-Röntgenbild und zugehörigem Soll-Röntgenbild;

Fig. 11 (a) bis 11 (c)      zeigen schematisch die Grundzüge einer pixelweisen Differenzbildung der numerischen Grauwerte zwischen Ist-Röntgenbild und zugehörigem Soll-Röntgenbild;

Fig. 12(a) und 12(b)      zeigen schematisch die Grundzüge des pixelweisen Schwellwertvergleichs und der Bestimmung zweidimensionaler Variationsgebiete aus den Differenzbildern;

Fig. 13(a) bis 13(c)      zeigen schematisch die Grundzüge der positionsbezogenen Relevanzprüfung der den zweidimensionalen Variationsgebieten zugrundeliegenden Dichtevariation;

Fig. 14      zeigt schematisch die Rückprojektion der zweidimensionalen Variationsgebiete in den dreidimensionalen CT-Datensatz des Patienten zur Bildung des dreidimensionalen Variationskörpers;

Fig. 15      zeigt schematisch den Zusammenhang zwischen Grauwert und Elektronendichte anhand eines eindimensionalen vereinfachten Beispiels;

Fig. 16      zeigt schematisch die Bestimmung von Einzelstrahlen des Bestrahlungsfeldes, die von der Dichtevariation betroffen sind; und

Fig. 17(a) bis 17(c)      zeigen verschiedene Möglichkeiten der Darstellung von Information zu der geänderten Reichweite des Bestrahlungsfeldes.

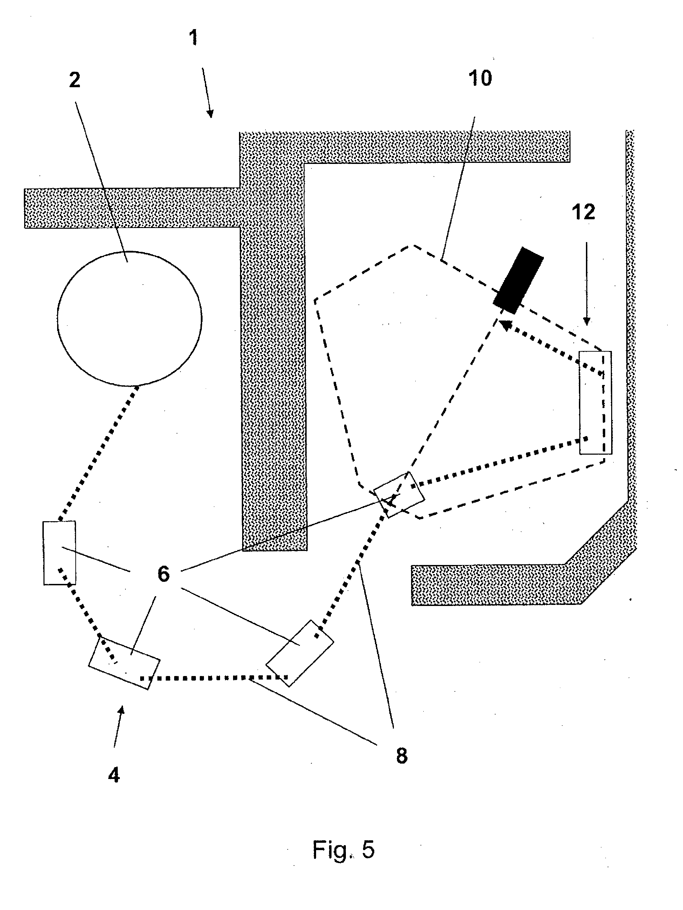

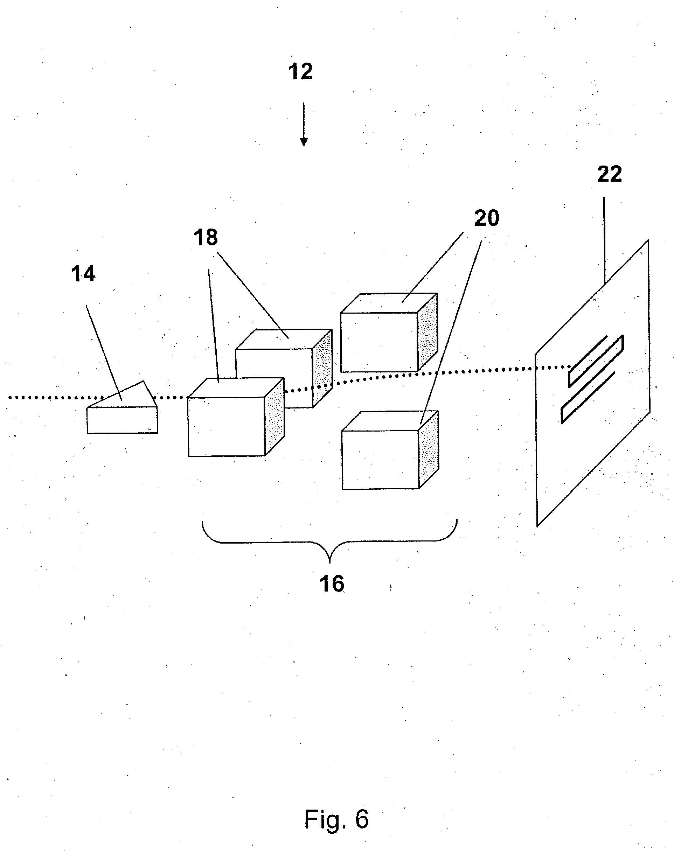

Um das in Fig. 3 dargestellte gleichmäßige oder auf gewünschte Weise anpassbare Bestrahlungsprofil eines Tumorgewebes im menschlichen Körper zu erzielen, umfasst die Anlage zur Bestrahlung von Patienten mit geladenen Teilchen zum Beispiel eine Raster-Scan-Bestrahlungseinheit 1, die schematisch in Fig. 5 dargestellt ist. Die Raster-Scan-Bestrahlungseinheit 1 weist einen Teilchenbeschleuniger 2 für geladene Teilchen auf. Als geladene Teilchen zur Bestrahlung von Tumoren kommen beispielsweise Protonen oder Schwerionen in Frage. Die Raster-Scan-Bestrahlungseinheit 1 weist außerdem eine Strahlführungseinheit 4 auf, die aus mehreren Strahlführungsmagneten 6 und zwischen diesen angeordneten, meist gerade verlaufenden Strahlführungsabschnitten 8 besteht. Die exakte Strahlführung ist eine der wichtigsten Herausforderungen bei der Bestrahlung mit geladenen Teilchen. Der Teilchenstrahl wird von der Strahlführungseinheit 4 in einen Behandlungsraum geleitet, in dem im vorliegenden Beispielsfall ein Gantry 10 angeordnet ist, welches sich um 360° drehen lässt. Das 3D-Scanning-System 12 dient zur zielgenauen Bestrahlung des Tumorgewebes und weist verschiedene, weiter unten in Bezugnahme auf Fig. 6 näher beschriebene Elemente zur Detailsteuerung des Teilchenstrahls auf.

Im dargestellten Beispielsfall ist die Ablenkeinheit 14 des 3D-Scanning-Systems 12 mit dem Ring des Gantrys 10 verbunden und kann gemeinsam mit diesem in eine beliebige Position gedreht werden, aus der der Patient bestrahlt wird. Hierdurch ist eine Bestrahlung aus verschiedenen Richtungen für verschiedene Applikationen möglich. Ebenso ist die Erfindung auf stationäre Bestrahlungsapparate anwendbar, die nicht als Gantry 10 ausgebildet sind.

Mit der Anlage werden die Patienten vorzugsweise in einem Raster-Scan-Verfahren bestrahlt, bei dem das Tumorgewebe in gleichmäßig beabstandete Rasterpunkte in einem dreidimensionalen Raster unterteilt wird, an denen eine Dosisapplikation stattfindet.

Um den Tumor auf gezielte Weise mit dem Behandlungsstrahl abzutasten bzw. zur genauen Ansteuerung der einzelnen 3D-Rasterpunkte im Zielvolumen besitzt das 3D-Scanning-System 12 (siehe Fig. 6) zunächst eine Energievariationseinheit 14 zur Einstellung der Energie des Teilchenstrahls und damit der Eindringtiefe des Strahls in den Patienten in Strahlrichtung. Im vorliegenden Beispiel ist die Energievariationseinheit 14 als Degrader-Keil ausgebildet, der jeweils über eine bestimmte Strecke in den Strahlengang geschoben werden kann und somit einen bestimmten Anteil der Energie des Teilchenstrahls absorbiert. Auf diese Weise ist die Energie des Teilchenstrahls und somit die Eindringtiefe des Teilchenstrahls in Strahlrichtung in den Körper submillimetergenau zu bestimmen. Neben einem Degrader-Keil sind auch andere Energievariationseinheiten 14 denkbar, beispielsweise Range Shifter-Platten.

Innerhalb einer jeden durch die Energievariationseinheit 14 definierten, quer zur Strahlrichtung angeordneten Schicht 22 vorgegebener Eindringtiefe im Patienten kann der Teilchenstrahl mittels einer Ablenkeinheit 16 gesteuert werden. Die Ablenkeinheit 16 weist beispielsweise einen zweipoligen Ablenkmagneten 18 zur Ablenkung des Teilchenstrahls in x-Richtung und einen zweipoligen Ablenkmagneten 20 zur Ablenkung des Teilchenstrahls in y-Richtung auf. Auch andere Ausgestaltungen sind denkbar. Die Ablenkeinheit 16 erzeugt somit in jeder Schicht 22 vorbestimmter Eindringtiefe ein mäanderndes Profil des Teilchenstrahls, wobei der Teilchenstrahl jeweils zwischen zwei Rasterpunkten je nach Punktabstand ausgeschaltet bleiben kann und erst nach vollständiger Einstellung der Ablenkmagnete 18, 20 auf den Tumor gelenkt wird, um eine präzise Dosisapplikation zu gewährleisten. Die Dosis bzw. die Anzahl von zu applizierenden geladenen Teilchen an benachbarten Rasterpunkten kann sich dabei je nach Form des Tumors erheblich unterscheiden.

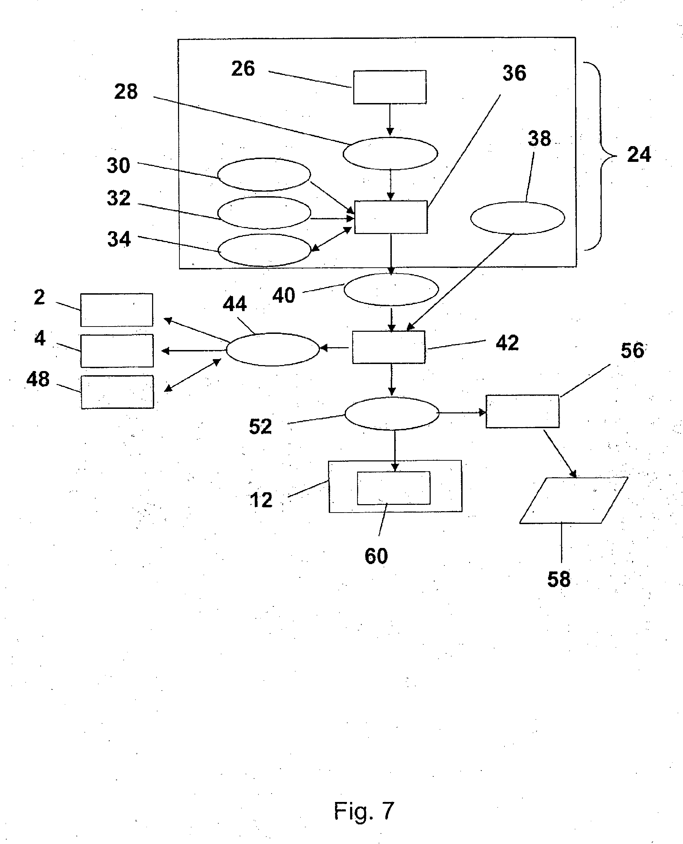

Für die Therapieplanung und die exakte Bestrahlung ist ein hochkomplexes System vorgesehen, welches die einzelnen Bestrahlungsparameter bis ins Kleinste festlegt und die Funktionalität der Anlage überwacht. Die elektronischen Komponenten einer Anlage, die an der Berechnung und Übertragung von Bestrahlungsdaten und Bestrahlungsbefehlen beteiligt sind, sind in Fig. 7 dargestellt. Das Therapieplanungssystem 24 umfasst eine Therapieplanungsstufe 26 zur Dosisberechnung und -optimierung. Wie bereits eingangs erläutert, fließen darin alle Ergebnisse der vorab durchgeführten medizinischen Beurteilung, Indikationsstellung, Zielvolumenkonturierung und des Therapiekonzepts sowie auch die dazugehörigen CT-Daten ein. Die Therapieplanungsstufe 26 berechnet daraus zunächst die gewünschte Dosisverteilung {x, y, z, Dosis} unter Berücksichtigung der vom zuständigen Arzt festgesetzten Parameter. Anschließend wird unter Einbeziehung der Funktionsdaten der Gesamtanlage daraus ein Therapieplan 28 generiert, der die aus der gewünschten Dosisverteilung berechnete Energie und Anzahl von geladenen Teilchen pro Einzelstrahl in jeder Schicht 22 festlegt. Somit wird jedem Einzelstrahl eine Information der Art {x, y, Energie der Teilchen, Anzahl der Teilchen} zugeordnet. Diese Daten des Therapieplans 28 werden beispielsweise an einen Therapiedatenspeicher 36 übermittelt, welcher zudem alle Patientendaten 30 und den dreidimensionalen CT-Datensatz 32 enthält. Außerdem sind an dieser Stelle Sicherheitseinrichtungen 34 vorgesehen, welche den Therapieplan genau überprüfen.

Das Therapieplanungssystem 24 stellt dann, beispielsweise aus dem Therapiedatenspeicher 36, alle Therapieplanungsdaten 40 dem Therapiekontrollsystem 42 bereit. Ebenso werden auch Konfigurationsdaten 38 der Anlage berücksichtigt, darunter bestimmte Maschineneinstellungen und -konfigurationen.

Das Therapiekontrollsystem 42 wandelt die vom Therapieplanungssystem 24 erzeugten Therapieplanungsdaten 40 in Bestrahlungsbefehle 44 für den Teilchenbeschleuniger 2 und die Strahlführungseinheit 4 um. Alle maschinenrelevanten Daten werden außerdem in hoch technisierten Sicherheitseinrichtungen 48 dauerhaft überprüft und überwacht. Dies reicht von der Überwachung der Türen über die Strahlüberwachung bis hin zur Überprüfung der Sensoren (nicht dargestellt), welche den Behandlungsstrahl während der Bestrahlung überprüfen und an sich schon eine eigene weitere Sicherheitseinrichtung darstellen.

Außerdem wandelt das Therapiekontrollsystem 42 die vom Therapieplanungssystem 24 erzeugten Therapieplanungsdaten 40 auch in Bestrahlungsdaten und Bestrahlungsbefehle 52 für das 3D-Scanning-System 12 um. Vorzugsweise ist im 3D-Scanning-System 12 ein Scanning-Steuermodul 60 enthalten, welches dazu geeignet ist, die vom Therapiekontrollsystem versandten Bestrahlungsdaten und Bestrahlungsbefehle 52 entgegenzunehmen und die Ansteuerung der Bestandteile des 3D-Scanning-Systems 12 auf dieser Basis zu übernehmen.

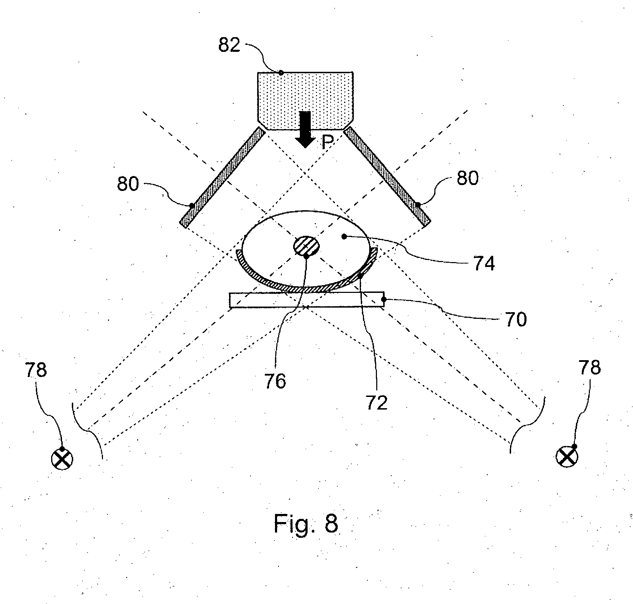

Eine Anordnung des Röntgensystems im Behandlungsraum für das erfindungsgemäße Verfahren zur in-situ Überwachung von Reichweiten-Unsicherheiten ist in Fig. 8 dargestellt. Auf dem Patiententisch 70 ist eine eigens für den Patienten 74 angefertigte Vakuummatratze 72 angeordnet, in welcher der Patient 74 während der Durchführung des Verfahrens und während der späteren Bestrahlung zu liegen kommt. Das Zielgebiet (Tumor) 76 ist ebenfalls skizziert.

Das Röntgensystem umfasst in der dargestellten Ausführungsform zwei Röntgenquellen 78, die mit zwei jeweils gegenüberliegend auf der anderen Seite des Patienten 74 angeordneten hochauflösenden Röntgendetektoren 80 zusammenwirken und somit zwei Projektionsrichtungen definieren. Am ergonomischsten und ökonomischsten ist sicherlich die Durchführung des Verfahrens mit einem Röntgensystem, bei dem die zwei Röntgendetektoren 80 seitlich symmetrisch zur Protoneneinstrahlrichtung P angeordnet sind. Grundsätzlich kann das beschriebene System jedoch mit beliebigen Winkeln der Röntgendetektoren 80 zur Protoneneinstrahlrichtung P angewandt werden. Vorzugsweise bewegen sich die Röntgendetektoren 80 und Röntgenquellen 78 bei der Drehung der Strahlvorrichtung 82 mit um den Patienten 74 herum. Die Röntgendetektoren 80 sind sogar vorzugsweise an der Strahlvorrichtung 82 befestigt. Es ist aber grundsätzlich auch denkbar, dass die Röntgendetektoren 80 und die Röntgenquellen 78 stationär im Behandlungsraum angeordnet sind.

In jedem Fall ist wichtig, dass der Zwischenwinkel zwischen den beiden Röntgendetektoren 80 zwischen 60° und 120° beträgt, bevorzugt zwischen 75° und 105°. Jeder Röntgendetektor 80 weist mindestens 500 x 500 Pixel auf, vorzugsweise mindestens 1.000 x 1.000 Pixel, mehr bevorzugt mindestens 2.000 x 2.000 Pixel. Die Abmessungen des Funktionsbereichs der Röntgendetektoren 80 sollten mindestens 20 x 20 cm betragen, vorzugsweise 30 x 30 cm, mehr bevorzugt 40 x 40 cm. Selbstverständlich können auch rechteckige Röntgendetektoren mit unterschiedlich langen Seiten eingesetzt werden.

In der Regel werden zwei orthogonale Röntgenprojektionen verwendet. In komplexeren Situationen kann das beschriebene System jedoch auch die Verrechnung von mehreren Projektionsrichtungen vorsehen, was die Zuverlässigkeit und das Auflösungsvermögen erhöht.

Die Anordnung und Größe sowie der Abstand zwischen Röntgenquelle 78 und Röntgendetektor 80 sollte möglichst so gewählt sein, dass in der Projektion der gesamte Patientenquerschnitt erfasst wird.

Dem beschriebenen System wird zunächst eine Reihe von Daten zur Verfügung gestellt. Diese sind sämtlich aus den ohnehin für die Therapieplanung erzeugten Daten extrahierbar. Im Einzelnen handelt es sich um folgende Daten:

- dreidimensionaler CT-Datensatz (hochaufgelöst, Schichtabstand z.B. 0,8 mm) für die betrachtete Körperregion (z.B. auf DICOM-Basis, einem medizinischen Standard)
- dreidimensionale sogenannte Structure-Sets (ebenfalls auf DICOM-Basis) des zu bestrahlenden Zielgebietes sowie der zu betrachtenden anderen Strukturen (z.B. gefährdete Organe, Rektumballon, etc.), welche dann auch für die Projektionen dienen
- eine geeignete Auswahl von Isodosis-Flächen, welche aus der Therapieplanung für das geplante Bestrahlungsfeld resultieren; diese werden ebenfalls als dreidimensionale Structure-Sets auf DICOM-Basis zur Verfügung gestellt
- eine Fluenz-Matrix, für die sich aus der Therapieplanung für das betrachtete Bestrahlungsfeld ergebenden Einzelstrahlen. Die Matrix enthält die lateralen Koordinaten des Einzelstrahls (x, y), die initiale Energie des Einzelstrahls (an der Patientenoberfläche), die vom Planungssystem errechnete Reichweite für diesen Einzelstrahl (z-Richtung) sowie die Fluenz des Einzelstrahls (d.h. die Anzahl von Protonen, welche zu einer Dosis beitragen sollen, angegeben typischerweise in Monitor Units (MU).

Nachdem sämtliche Daten für den aktuell zu behandelnden Patienten 74 in das System geladen wurden, wird im ersten Schritt der Patient 74 auf dem Patiententisch 70 positioniert und seine Lage dann genau überprüft. Dies erfolgt mit einem unabhängigen und hier nicht vorgestellten System (Positionsverifikationssystem).

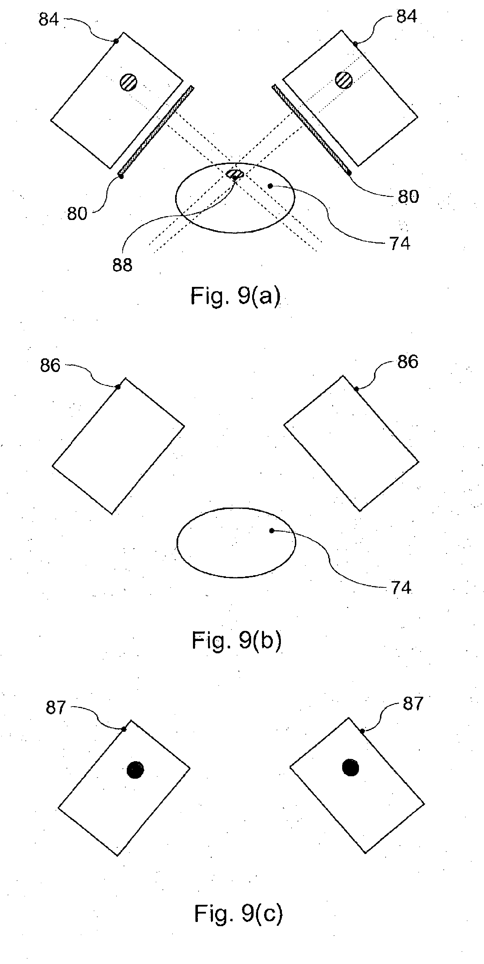

In der korrekten Patientenposition werden mit Hilfe des Röntgensystems zwei Ist-Röntgenbilder 84 (siehe Fig. 9(a) mit umgeklappter Darstellung der Ist-Röntgenbilder 84 der Röntgendetektoren 80), also Projektionsaufnahmen des relevanten Bereiches des Patienten 74 um das Zielgebiet 76 des Protonenstrahls aufgenommen. Des Weiteren werden aus dem dreidimensionalen CT-Datensatz 32 entsprechende digitale rekonstruierte Soll-Röntgenbilder 86 (sog. DRR = digitale rekonstruiertes Röntgenbilder) für die zum Röntgensystem identischen Projektionsrichtungen erzeugt, siehe Fig. 9(b). Digitale rekonstruierte Röntgenbilder ähneln normalen Röntgenbildern, stammen aber aus den Bilddaten einer Computertomographie. Im Computer wird eine virtuelle Sicht auf den dreidimensionalen Datensatz berechnet, wobei die Voxel eine dichteabhängige Transparenz erhalten. Das Ergebnis ist ein Summationsbild einer in Blickrichtung gelegenen Struktur. Dabei wird aus dem CT-Datensatz ein Bild mit der gleichen geometrischen Perspektive wie die Röntgendetektoranordnung erzeugt. Das Bild entsteht durch eine Voxel-Summation entlang des sich vom virtuellen Fokus zur virtuellen Bildebene ausbreitenden divergenten Strahlenbündels. Hierfür werden bekannte Ermittlungsverfahren verwendet.

Anschließend wird eine Differenzanalyse zur Feststellung von Unterschieden zwischen Ist-Röntgenbild 84 und zugehörigem Soll-Röntgenbild 86 für die zwei Projektionsrichtungen durchgeführt. Ohne Vorliegen einer Dichtevariation würden Ist-Röntgenbild 84 und zugehöriges Soll-Röntgenbild 86 keine Unterschiede zeigen. In dem in Fig. 9(a) skizzierten schematischen Fall eines homogenen Körpers und einer homogenen Dichtevariation 88 ergibt sich dann im Differenzbild 87 (Fig. 9(c)) ein klares Signal für den Dichteunterschied.

Für diese Differenzanalyse können einige Vorbereitungen notwendig sein. Zum Beispiel wird, falls notwendig, der Bereich sowohl in den Soll-Röntgenbildern 86 als auch in den Ist-Röntgenbildern 84 ausgewählt, welcher der Schnittmenge zwischen beiden in der jeweiligen Projektion entspricht. Danach kann u.U. die Auflösung durch geeignete Bildskalierungsalgorithmen auf die höhere von beiden, also entweder auf die Auflösung der Ist-Röntgenbilder 84 oder auf die Auflösung der Soll-Röntgenbilder 86 angepasst werden.

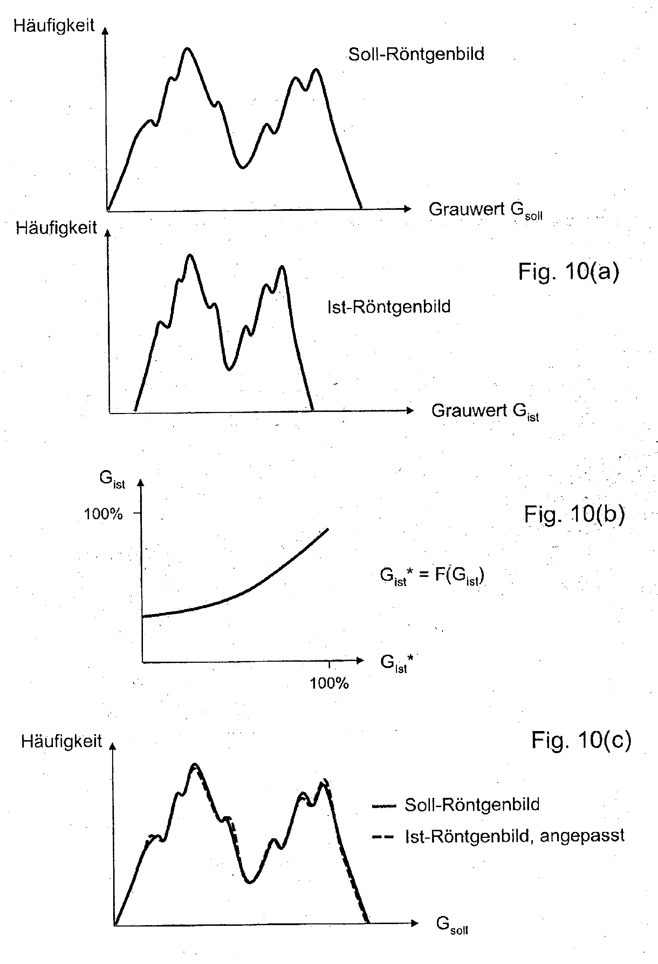

Außerdem kann eine Grauwerteanpassung der Ist-Röntgenbilder 84 und der Soll-Röntgenbilder 86 notwendig sein. Die Grundzüge einer Grauwerteanpassung sind in Fig. 10(a) bis 10(c) dargestellt.

Die Ausgangssituation bei der Grauwerteanpassung ist eine bestimmte Grauwerteverteilung im Soll-Röntgenbild 86. Bei identischem durchleuchteten Objekt und identischer dynamischer Abbildung in Grauwerte ist das Ist-Röntgenbild 84 identisch zum Soll-Röntgenbild 86. Meist werden sich jedoch Unterschiede ergeben, da das Röntgensystem gerätespezifisch ein anderes dynamisches Verhalten zeigt wie das virtuelle Soll-Röntgenbild 86, das aus dem CT-Datensatz 32 erzeugt wurde. Trägt man die Histogramme der Grauwerte für Soll- und Ist-Röntgenwert auf, so sollte bei identischem Objekt auch die Grauwerteverteilung im Histogramm nach ihrer Häufigkeit identisch sein. Ist die zu identifizierende Dichtevariation 88 nicht zu groß (beispielsweise über den gesamten Abbildungsbereich) so sind die Unterschiede in den Grauwertehistogrammen zu vernachlässigen. Dies gilt insbesondere, da die Grauwerteverteilung dominiert wird von den anatomischen Strukturen, wie Knochen und Weichteilgewebe. Es ist deshalb als Annäherung zulässig, das Ist-Röntgenbild 84 in seiner Grauwerteverteilung an die Grauwerteverteilung des Soll-Röntgenbildes 86 anzupassen.

Fig. 10(a) zeigt die Ausgangssituation mit den beiden Grauwerteverteilung für das Soll-Röntgenbild 86 und das Ist-Röntgenbild 84. Gezeigt ist hier schematisch, dass das Ist-Röntgenbild 84 dieselbe Information (Form der Verteilung) enthält, aber die Dynamik anders ist. Mit einer Anpassungsfunktion (Fig. 10(b)) wird das Ist-Röntgenbild 84 nun an das Soll-Röntgenbild 86 angepasst. Diese Anpassungsfunktion F ist im einfachsten Fall ein Polynom zweiten Grades. Je nach verwendetem Gerätetyp bei der Anordnung kann dieses Polynom zweiten Grades durch eine andere Funktion ersetzt werden. In einem iterativen numerischen Optimierungsverfahren werden nun die bestimmenden Parameter für die Anpassungsfunktion ermittelt, so dass die angepasste Grauwerteverteilung des Ist-Röntgenbildes 84 möglichst gut mit der Grauwerteverteilung des Soll-Röntgenbildes 86 übereinstimmt (Fig. 10(c)). Optimiert wird hierbei auf eine $x^2$-Funktion, welche die jeweilige Differenz der beiden Häufigkeiten in den Grauwerte-Histogrammen bei den einzelnen Grauwerten beinhaltet. Die Optimierungsstrategie sieht dabei vor, dass zuerst die niederen Grade der Anpassungsfunktion variiert werden, da diese die physikalischen Unterschiede zwischen Ist-Röntgenbild 84 und Soll-Röntgenbild 86 maßgeblich abbilden.

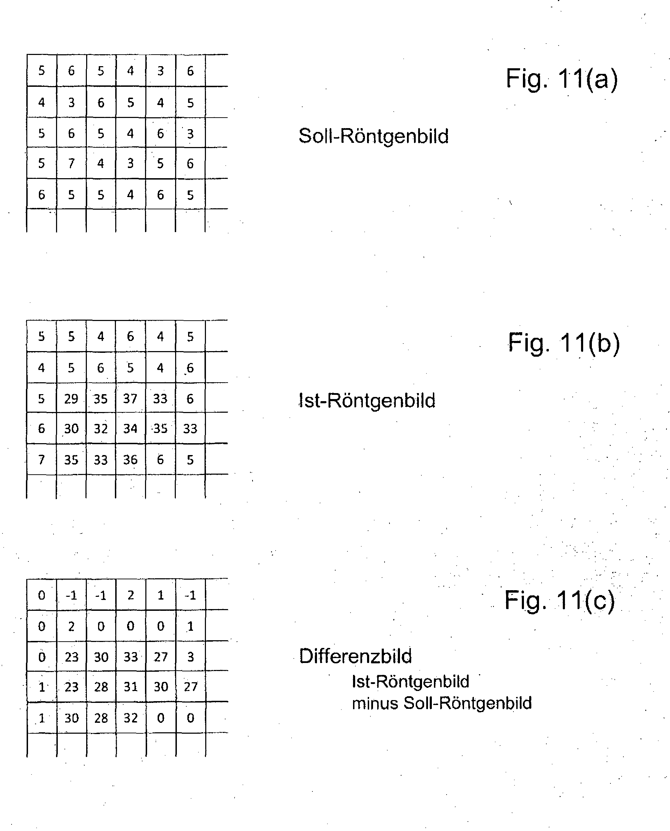

Da nun sowohl das Soll-Röntgenbild 86 als auch das Ist-Röntgenbild 84 in der gleichen Auflösung und in dem gleichen Grauwertebereich vorliegen, kann, wie in Fig. 11 (a) bis 11 (c) dargestellt, eine pixelweise Differenzbildung der numerischen Grauwerte erfolgen. Dabei können sowohl negative als auch positive Grauwertdifferenzen auftreten, je nach dem, ob eine Dichtevariation 88 mit erhöhter Dichte im detektierten Bereich oder mit einer erniedrigten Dichte im Vergleich zur ursprünglichen Dichteverteilung vorliegt.

Neben der hier beschriebenen Methode existieren weitere Möglichkeiten der Differenzanalyse zwischen Ist-Röntgenbild 84 und zugehörigem Soll-Röntgenbild 86, die hier nicht näher beschrieben werden sollen.

Im nächsten Schritt ist es gewünscht, die Relevanz der festgestellten Unterschiede bei der Differenzanalyse hinsichtlich räumlicher Ausdehnung und hinsichtlich Lokalisation in Bezug auf das geplante Bestrahlungsfeld zu überprüfen und relevante zweidimensionale Variationsgebiete festzulegen. Auch hierfür existiert eine Vielzahl von Möglichkeiten. Im Folgenden soll eine besonders vorteilhafte Methode beschrieben werden.

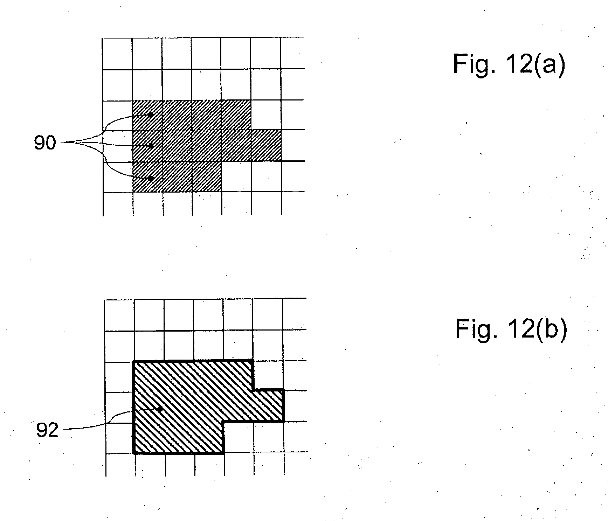

Wenn wir wieder zum Ergebnis aus Fig. 11 (c) zurückkehren, kann aufgrund begrenzter örtlicher Auflösung sowie von Streu- und Rauscheffekten auch in den Differenzbildern ein Rauschen auftreten. Deshalb kann zunächst ein Erkennungsalgorithmus angewendet werden, welcher entscheidet, ob eine maßgebliche Grauwertvariation vorliegt. Hierzu wird zunächst für jedes Pixel überprüft, ob ein bestimmter Schwellwert für positive Grauwertsdifferenzen überschritten wurde bzw. ein negativer Schwellwert für negative Grauwertdifferenzen. Die in unserem Ausführungsbeispiel vorliegenden Pixel 90 oberhalb des Schwellwerts sind in Fig. 12(a) dargestellt. Die Schwellwerte sind durch entsprechende Rauschwertermittlungsverfahren für das individuelle Röntgensystem zu ermitteln. Die Abhängigkeit der Schwellwerte von den verwendeten Röntgenparametern (mAs, kV) wird ebenfalls in der Rauschwertermittlungsprozedur berücksichtigt.

Als nächstes werden zusammenhängende Gebiete von Pixeln 90 ermittelt, welche eine Grauwertvariation über dem Schwellwert aufweisen. Diese Gebiete werden im Folgenden als zweidimensionale Variationsgebiete 92 bezeichnet, siehe Fig. 12(b). Hier kann die Sensibilität bezüglich der Ausdehnung solcher zweidimensionaler Variationsgebiete 92 vom klinischen Benutzer festgelegt werden, indem eine Mindestanzahl von Pixeln 90 in einem solchen zusammenhängenden Gebiet definiert wird oder die Flächenausdehnung in Form von minimaler Breite und Höhe vorgegeben wird.

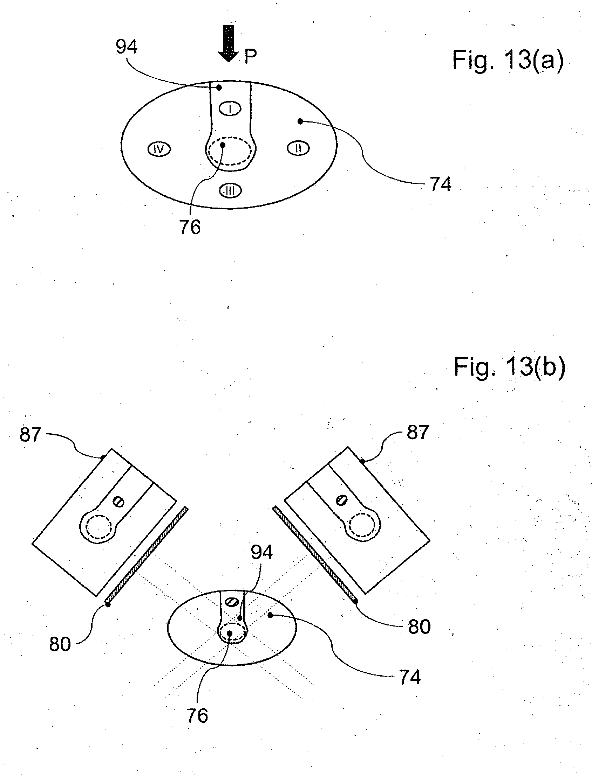

Im nächsten Prozessschritt erkennt das System, ob die dem ermittelten zweidimensionalen Variationsgebiet 92 zugrundeliegende Dichtevariation im relevanten Einstrahlbereich des Protonenstrahles für das geplante Bestrahlungsfeld liegt und deshalb überhaupt zu einer Reichweitenvariation beitragen kann. Eine solche Identifikation ist aus einer Projektionsrichtung nicht möglich, weshalb die Erfindung immer eine zweite Projektionsrichtung vorsieht. Fig. 13(a) zeigt eine schematisierte Situation. Gezeichnet ist der schematische Querschnitt des Patienten 74 mit dem Zielgebiet 76 der Protonenbestrahlung. Ebenfalls gezeigt sind der Protoneneinstrahlbereich 94 und mögliche Positionen I, II, III und IV von Dichtevariationen. Mit Hilfe der zuvor beschriebenen DICOM-Structure-Sets für Zielgebiet, gefährdete Organe, sonstiger Strukturen sowie den Isodosisflächen können nun diese Strukturen entsprechend in die Differenzbilder 87 projiziert werden. Dies ist schematisch in Fig. 13(b) dargestellt.

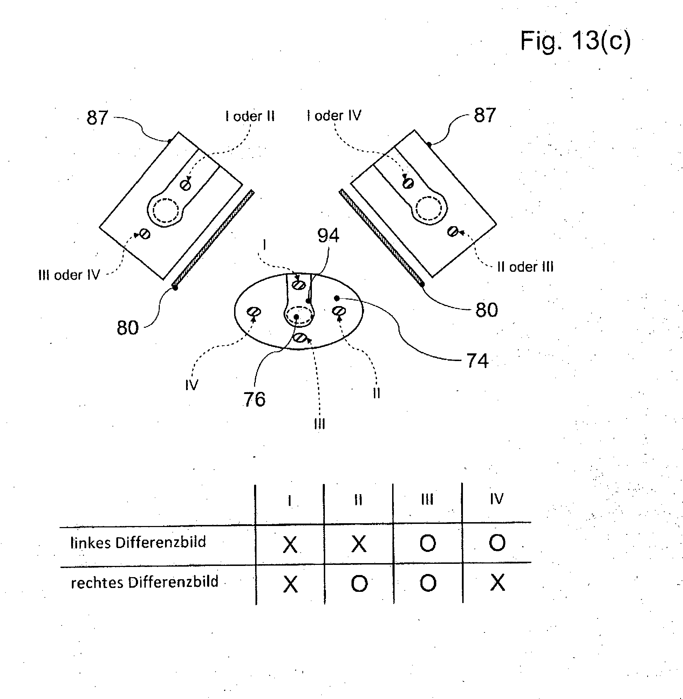

Die räumliche Zuordnung der Dichtevariation, die den identifizierten zweidimensionalen Variationsgebieten 92 zugrunde liegt, erfolgt nun über die Auswertung der Lokalisation in den beiden Projektionsrichtungen. Fig. 13(c) zeigt die möglichen Differenzbilder 87 in den beiden Projektionsrichtungen und eine Tabelle, in der für jede mögliche Position I, II, III und IV der Dichtevariation im Patienten 74 für jedes Differenzbild 87 eingetragen ist, ob diese Dichtevariation im Einstrahlbereich 94 liegen kann (x) oder nicht (o). Durch Überlagerung der Informationen beider Projektionsrichtungen gelangt man im vorliegenden Fall zum Ergebnis, dass nur bei Position I beide Projektionsrichtungen eine mögliche Relevanz erkennen lassen, während die Positionen II, III und IV als nicht relevant ausgeschlossen werden können, weil die Dichtevariation an dieser Stelle für eine Reichweitenänderungsbetrachtung nicht relevant ist.

Das beschriebene System kann diese Information visuell für den klinischen Benutzer in den beiden Differenzbildern 87 darstellen, indem z.B. die zweidimensionalen Variationsgebiete 92 durch unterschiedliche farbliche Umrandung als relevant oder irrelevant gekennzeichnet werden. Die Auswerteeinheit kann aber auch einfach nur positionsirrelevante zweidimensionale Variationsgebiete 92 ausblenden und für die weitere Berechnung vernachlässigen.

Für eine Schnellauswertung der Reichweitenverschiebung wird vom beschriebenen System als nächstes eine Mittelung der Grauwerte in den Variationsgebieten 92, welche reichweitenrelevant sind, durchgeführt. Bestimmt werden die Mittelwerte für die beiden Ist-Röntgenbilder 84 sowie die beiden Soll-Röntgenbilder 86. Jeweils wird eine für das spezifische System ermittelte Rauschkorrektur z.B. in Form eines Abzugs durchgeführt. Es ergeben sich die vier Graumittelwerte für die vier Bilder.

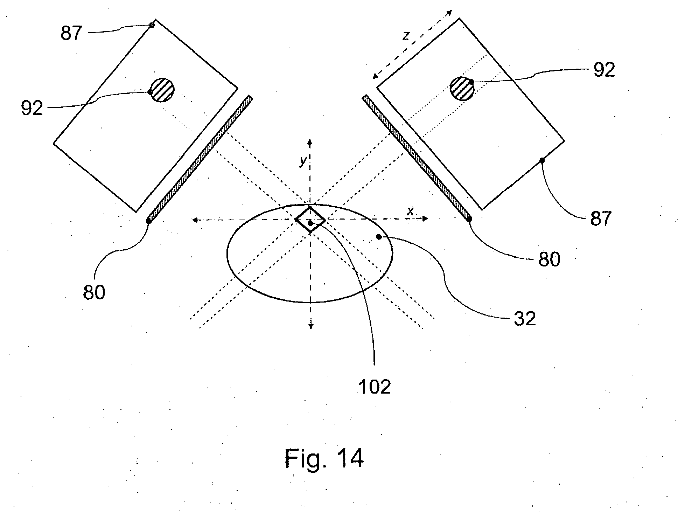

In Weiterführung bestimmt das System nun die genäherte räumliche Ausdehnung und räumliche Position der Dichtevariation, die den relevanten zweidimensionalen Variationsgebieten 92 zugrund liegt. Hierzu wird eine Rückprojektion der zweidimensionalen Variationsgebiete 92 aus den Differenzbildern 87 in den dreidimensionalen CT-Datensatz 32 des Patientenkörpers durchgeführt. Da dies für zwei Richtungen durchgeführt wird, ergibt sich an der Position $X_v$, $y_v$, $Z_v$ ein Schnittkörper, im folgenden dreidimensionaler Variationskörper 102 genannt (siehe Fig. 14). Hierbei sind die Koordinaten für die weitere Beschreibung so festgelegt, dass die z-Richtung in Körperlängsachse bei liegendem Patienten weist und dann x und y ein Rechtskoordinatensystem bilden, wobei x in der Horizontalenebene liegt. Dabei wird deutlich, dass in x- und y-Richtung durch die Rückprojektion von zwei Aufnahmen lediglich ein rautenförmiges Gebiet identifiziert werden kann. Das hier beschriebene System unterscheidet deshalb mehrere Detaillierungsstufen, die vom klinischen Personal in der vorliegenden Situation gewählt werden können.

Im einfachsten Fall wird die dreidimensionale Form des Variationskörpers 102 durch eine Vereinfachung der zweidimensionalen Variationsgebiete 92 in Form von Rechtecken mit der mittleren Breite und mit der mittleren Länge der zweidimensionalen Variationsgebiete 92 und den daraus folgenden Schnittflächen im 3D-Volumen abgebildet. Es entsteht ein Polyhedron. Dies reicht im Weiteren für eine sehr schnelle Rekonstruktion und Abschätzung einer möglichen Reichweitenvariation aus.

Die zweite Näherungsstufe besteht darin, dass unter der Annahme, dass in der Regel in einem menschlichen Körper keine strikten eckigen Strukturen vorkommen, die in Fig. 14 dargestellte Querschnittsfläche durch eine Ellipse angenähert wird und somit anatomische Strukturen besser annähert. Die Längenausdehnung wird dennoch über die mittlere Länge in z-Richtung des zweidimensionalen Variationsgebiets 92 bestimmt. Dadurch ergibt sich ein zylinderförmiges Volumen für den Variationskörper 102 mit der Dichtevariation im 3D-Datensatz.

Eine weitere Näherungsmöglichkeit, die das System zur Verfügung stellt, ist die Annahme von elliptischen Konturen in jeweils beiden Projektionen sowie im Schnittbild, wodurch sich ein Ellipsoid als dreidimensionale Form ergibt.

Darüberhinaus stellt das System die Näherungsmöglichkeit zur Verfügung, bei der für jede x,y-Projektionsschnittebene die tatsächlichen Ausdehnungen der zweidimensionalen Variationsgebiete 92 in der Schnittebene verwendet werden. Dadurch ergibt sich durch die Rückprojektion für jede z-Position im CT-Datensatz ein rautenförmiges Gebiet wie in Fig. 14 skizziert, jedoch mit gegebenenfalls unterschiedlicher Ausdehnung. Dieser "Stapel" von rautenförmigen Gebieten ergibt dann durch Interpolation in z-Richtung zwischen den z-Positionen den dreidimensionalen Variationskörper 102. Das System bietet auch die Möglichkeit, die beschriebenen rautenförmigen Gebiete durch Ellipsen anzunähern, wodurch sich als dreidimensionaler Variationskörper 102 ein "wurstförmiges" Volumen ergibt.

Durch zusätzliche Projektionsrichtungen, die das System grundsätzlich ebenfalls erlaubt, kann eine verbesserte Konturfindung erfolgen und so die dreidimensionale Ausformung des Variationskörpers 102 besser beschrieben werden.

Nachdem nun dem System hinreichend viele Informationen vorliegen, kann durch Lösen eines Integralgleichungssystems auf die tatsächliche Dichtevariation im Variationskörper 102 geschlossen werden und in den darauffolgenden Prozessschritten die Auswirkung auf die Reichweite der Protonenstrahlen bestimmt werden.

Dieses Integralgleichungssystem kann in jedem Fall numerisch gelöst werden. Eine numerische Lösung mit höherem Detaillierungsgrad erfordert dabei einen erhöhten Rechen- und Zeitaufwand.

Dem System steht zu diesem Zeitpunkt der vollständige 3D-Datensatz 32 aus der Computertomographie für den interessierenden Körperabschnitt des Patienten zur Verfügung. Dieser enthält sowohl die Houndsfield-Units-Verteilung, die Elektronendichteverteilung p (x, y, z) und die entsprechende Zuordnung zum Bremsvermögen von Protonen dE/dx (x, y, z, E). Des Weiteren die identifizierte Lage und Ausdehnung des Variationskörpers 102 mit der Dichtevariation im Körper und die Entscheidung, ob der Variationskörper 102 im Einstrahlbereich 94 des Protonenstrahlenfeldes liegt. Außerdem liegen die gemittelten Grauwerte der identifizierten zweidimensionalen Variationsgebiete 92 in den Ist-Röntgenbildern 84 und den Soll-Röntgenbildern 86 für beide Projektionsrichtungen vor.

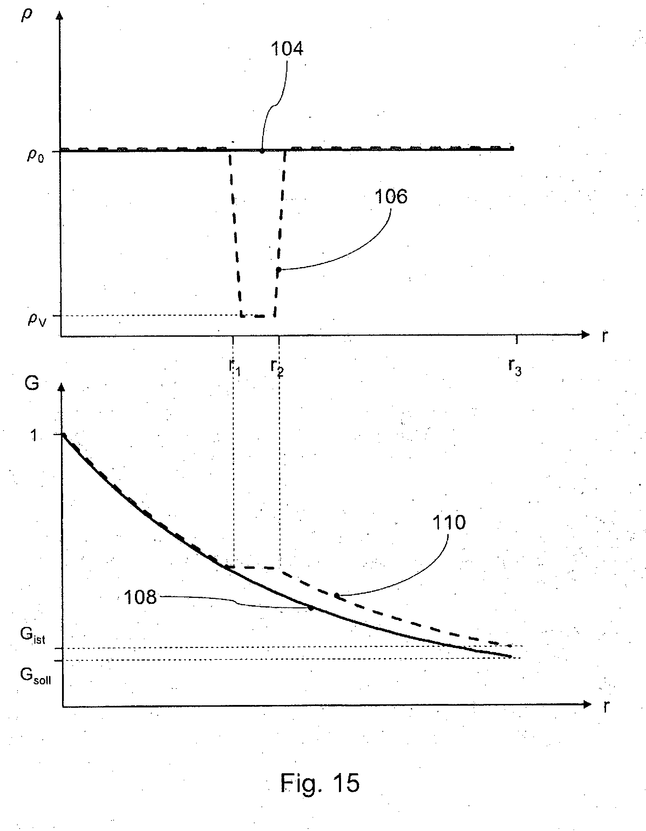

Auf Basis dieser Information kann nun, wie in Fig. 15 skizziert, zu jeder Projektionsrichtung ein Gleichungssystem aufgestellt und gelöst werden, das die mittlere Elektronendichte p der gesuchten Dichtevariation zur Lösung hat. Dabei wird der grundlegende Zusammenhang

$$G(r) = e^{-\alpha \cdot \rho \cdot r}$$

verwendet, wobei G der numerische Grauwert, $\alpha$ eine Konstante und p die Elektronendichte ist.

In Fig. 15 ist dies zur Veranschaulichung für den einfachsten Fall von homogenen Dichtebereichen für eine Dimension dargestellt. Im oberen Diagramm der Fig. 15 bezeichnet die durchgezogene Linie 104 den Fall einer homogenen Elektronendichteverteilung, während die gestrichelte Linie 106 den Fall einer homogenen Elektronendichteverteilung mit einem homogenen Variationskörper 102 darstellt. Im unteren Diagramm werden entsprechende Grauwertekurven des homogenen Falls 108 und des Falls 110 mit dem Variationskörper entsprechend dargestellt.

Das zugehörige Gleichungssystem für diesen Fall kann folgendermaßen skizziert werden:

$$G_{Soll} = e^{-\rho_0 \cdot r_3}$$

$$G_{Ist} = e^{-\rho_0 \cdot r_1} \cdot e^{-\rho_v \cdot (r_2 - r_1)} \cdot e^{-\rho_0 \cdot (r_3 - r_2)}$$

$$\frac{G_{Soll}}{G_{Ist}} = g = e^{(\rho_0 - \rho_v) \cdot (r_1 - r_2)}$$

$$\Rightarrow \rho_v = \rho_0 + \frac{\ln g}{r_2 - r_1}$$

Dieses vereinfacht dargestellte Gleichungssystem kann auf sämtliche notwendigen Dimensionen verallgemeinert werden und mindestens numerisch gelöst werden.

Das System hat in der Prozesskette nun den mittleren Elektronendichtewert des identifizierten Variationskörpers 102 in Ort, Ausdehnung und Größe bestimmt. Aus der Therapieplanung und den zugrundeliegenden Kalibrierungen für das spezifische System kann nun direkt das Bremsvermögen für Protonenstrahlen bzw. dessen Verteilung hieraus bestimmt werden.

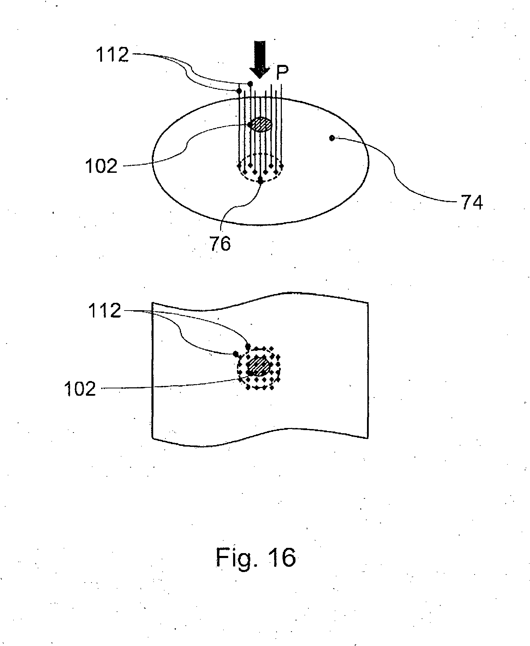

Hierzu identifiziert das System zunächst die von der Dichtevariation des Variationskörpers 102 betroffenen Einzelstrahlen 112 des geplanten Bestrahlungsfeldes. Fig. 16 zeigt schematisch das Vorgehen. Zu erkennen ist im Querschnitt sowie in Draufsicht aus Protoneneinstrahlrichtung der identifizierte Variationskörper 201 sowie die Strahllagen der Einzelstrahlen des geplanten Protonenbestrahlungsfeldes und deren ursprüngliche Reichweite. Da die Fluenz-Matrix der Einzelstrahlen 112 aus der Therapieplanung bekannt ist und dem beschriebenen System zur Verfügung gestellt wurde und die dreidimensionale Ausdehnung des Variationskörpers 102 bekannt ist, wird das System durch einfache Schnittbildung die Einzelstrahlen 112 identifizieren, die von der Richtung her durch den Variationskörper 102 dringen und deren ursprüngliche Reichweite gleichzeitig im Bereich des Variationskörpers 102 oder dahinter gelegen hätte.

Das System kennt nun die betroffenen Einzelstrahlen 112 der originären Fluenz-Matrix und die Elektronendichte im Variationskörper 102. Für jeden betroffenen Einzelstrahl 112, dessen Richtung und Position dem System bekannt ist, berechnet das System die Reichweite neu. Die Berechnung der Reichweite der Einzelstrahlen 112 erfolgt analog zum Therapieplanungssystem. Jedoch wird die Dichteverteilung im Bereich des identifizierten Variationskörpers 102 durch die ermittelte mittlere Elektronendichte in diesem Bereich ersetzt. In den anderen Bereichen des Körpers wird die Dichteverteilung, wie sie aus dem CT-Datensatz 32 bekannt ist, unverändert verwendet. Der grundlegende Zusammenhang zwischen Elektronendichte und Reichweite des Protonenstrahls ist Fig. 4(a) und 4(b) zu entnehmen.

Die Umrechnung der Dichtewerte p(x) in den verschiedenen Voxeln in das Bremsvermögen für Protonenstrahlen dE/dx(x) findet gemäß der für das spezifische CT ermittelten Kalibrierungsverfahren statt. Die Reichweite R des Einzelstrahls 112 wird über seinen differentiellen Energieverlust entlang seines Eindringpfades bestimmt. Die Reichweite entlang der Einzelstrahlrichtung ist dabei das Integral über den energieabhängigen differentiellen Energieverlust, bis die Energie des Einzelstrahls 112 auf thermische Energiewerte (< 10 eV) reduziert ist.

In der nachfolgenden Gleichung wird für den homogenen Fall (konstanter Dichtewert) der grundlegende Zusammenhang dargestellt.

$$R = \int_{E_{therm}}^{E_0} \left( \frac{1}{\rho} \cdot \frac{dE}{dx} \right)^{-1} dE$$

wobei:

$E_0$ = initiale Energie
$E_{therm}$ = finale Energie ($\leq$10 eV)

$\rho$ Dichte in g/$_{cm}$$^3$

dE/dx = differentieller Energieverlust = Bremsvermögen in MeV/cm

Im beschriebenen System wird die Reichweite der Einzelstrahlen numerisch bestimmt.

Das beschriebene System stellt nun dem klinischen Benutzer eine Reihe von Darstellungsvarianten zur Verfügung, nach denen der klinische Benutzer entscheiden kann, ob die identifizierte Reichweitenvariation klinisch relevant ist oder nicht:

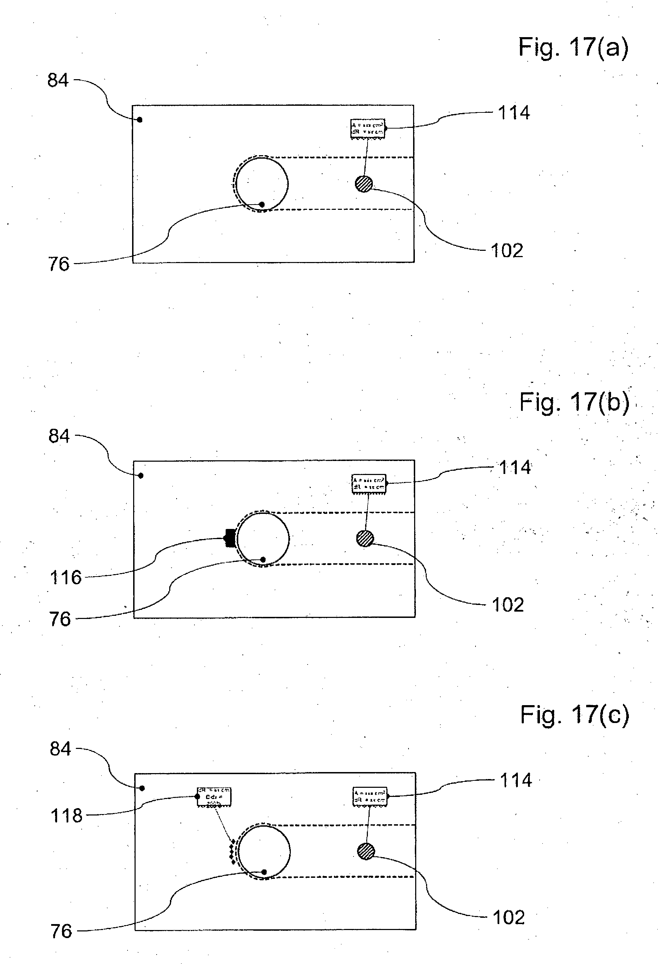

a) In einer in Fig. 17(a) skizzierten ersten Darstellungsvariante wird im Ist-Röntgenbild 84 (und/oder im Soll-Röntgenbild 86) ein einfacher Warnhinweis 114 ausgegeben, indem visuell in der Projektion der identifizierte Variationskörper 102 mit den Angaben über rechnerisch abgeschätzte Reichweitenvariationen in cm und Ausdehnung der Reichweitenvariation in cm$^2$ versehen wird. In dieser einfachen und besonders schnellen Variante kann ggf. nur auf den Teil der Einzelstrahlen 112 zugegriffen werden, welcher die höchste ursprüngliche Reichweite am jeweiligen Ort (x,y) des Bestrahlungsfeldes betrifft (Distal-Edge), denn diese tragen in der Regel auch den höchsten Dosisbeitrag und führen zu der relevantesten klinischen Auswirkung einer Reichweitenvariation.

b) Zusätzlich zur genannten Darstellungsvariante a) kann in den beiden Ist-Röntgenbildern 84 (und/oder Soll-Röntgenbildern 86) der Bereich 116 farblich markiert werden, welcher einer Reichweitenvariation unterliegt. Dies ist in Fig. 17(b) für den Fall einer Reichweitenerhöhung durch eine Dichtevariation mit erniedrigter Dichte im Protoneneinstrahlbereich schematisch dargestellt.

c) In einer weiterführenden Variante wird vom System für jeden einzelnen der in den beiden oben genannten Varianten verwendeten Distal-Edge-Einzelstrahlen 112 die Reichweitendifferenz berechnet und deren neue Reichweitenendpunkte analog zu Variante (b), aber für jeden Einzelstrahl 112 einzeln dargestellt, gegebenenfalls mit einer Vergrößerungsfunktion bei Auswahl eines Einzelstrahls 112. Zusätzlich kann für diese Einzelstrahlen 112 jeweils in einem Feld 118 ein Wichtungsindex angegeben werden, der darstellt, wie groß der Beitrag dieses Einzelstrahls 112 zur Dosisverteilung jeweils im Vergleich zum mittleren Beitrag aller Einzelstrahlen 112 ist. Der klinische Benutzer des Systems kann hieraus eine detailliertere Entscheidung treffen.

d) Die Variante (c) wird auf eine vom Benutzer wählbare Anzahl von Energieschichten im Gegensatz zu lediglich der tiefsten Energieschicht (Distal-Edge) erweitert (nicht dargestellt).

e) Durch Neuberechnung der Reichweite für sämtliche Einzelstrahlen 112 der gesamten Fluenz-Matrix und der Verwendung eines Dosisalgorithmus analog zum Therapieplanungssystem kann vom System in der höchsten Detaillierungsstufe eine neue Isodosisverteilung berechnet und in den beiden Projektionsdarstellungen der Ist-Röntgenbilder 84 und/oder Soll-Röntgenbilder 86 visualisiert werden (nicht dargestellt).

## Patentansprüche

1. Verfahren zur Identifikation möglicher Veränderungen der Reichweite eines geplanten Bestrahlungsfeldes vor der Bestrahlung eines Patienten (74) mit geladenen Teilchen, mit folgenden Schritten:

Durchführen einer Computertomographie über zumindest einen Teil des Patienten (74) zur Erzeugung eines dreidimensionalen Computertomographiedatensatzes (32);
Bereitstellen eines Röntgensystems aus zwei hochauflösenden Röntgendetektoren (80), die miteinander einen Winkel von 60° bis 120°, bevorzugt von 75° bis 105°, einschließen, und zwei den Röntgendetektoren (80) gegenüberliegenden Röntgenquellen (78), wobei jedes Paar von Röntgenquelle (78) und Röntgendetektor (80) jeweils eine Projektionsrichtung definiert;
Erstellen von zwei Ist-Röntgenbildern (84) zumindest eines Teils des Patienten (74) in den zwei Projektionsrichtungen mit den zwei Röntgendetektoren (80);
Berechnen von zwei digitalen rekonstruierten Soll-Röntgenbildern (86) aus dem Computertomographiedatensatz (32) entsprechend den zwei Projektionsrichtungen des Röntgensystems;
Durchführen einer Differenzanalyse zur Feststellung von Unterschieden zwischen Ist-Röntgenbild (84) und zugehörigem Soll-Röntgenbild (86) für die zwei Projektionsrichtungen;
Überprüfen der Relevanz der festgestellten Unterschiede hinsichtlich räumlicher Ausdehnung und hinsichtlich Lokalisation in Bezug auf das geplante Bestrahlungsfeld und Festlegen von relevanten zweidimensionalen Variationsgebieten (92);

Festlegen eines dreidimensionalen Variationskörpers (102) durch Rückprojektion der zweidimensionalen Variationsgebiete (92) in den dreidimensionalen Computertomographiedatensatz (32);
Berechnen einer mittleren Elektronendichte (p) des dreidimensionalen Variationskörpers (102) auf Basis der festgestellten Unterschiede zwischen Ist-Röntgenbild (84) und zugehörigem Soll-Röntgenbild (86) für die zwei Projektionsrichtungen;
Identifizieren von Einzelstrahlen (112) des geplanten Bestrahlungsfeldes, die durch den dreidimensionalen Variationskörper (102) treten;
Berechnen von zu erwartenden geänderten Reichweiten der identifizierten Einzelstrahlen (112) des geplanten Bestrahlungsfeldes unter Berücksichtigung des geänderten Elektronendichtewerts (p) im dreidimensionalen Variationskörper (102); und
Darstellen oder Weiterleiten von Informationen (114, 116, 118) über die berechneten geänderten Reichweiten.

2. Verfahren nach Anspruch 1, **dadurch gekennzeichnet, dass** der Schritt des Durchführens einer Differenzanalyse zur Feststellung von Unterschieden zwischen IstRöntgenbild (84) und zugehörigem Soll-Röntgenbild (86) den Schritt umfasst, numerische Grauwerte von sich entsprechenden Pixeln (90) des Ist-Röntgenbildes (84) und Soll-Röntgenbildes (86) zu subtrahieren, um eine pixelweise Grauwertabweichung zu bestimmen.

3. Verfahren nach Anspruch 2, **dadurch gekennzeichnet, dass** vor dem Schritt des pixelweisen Subtrahierens der numerischen Grauwerte eine Grauwertanpassung zwischen den Ist-Röntgenbildern (84) und den Soll-Röntgenbildern (86) durchgeführt wird.

4. Verfahren nach Anspruch 3, **dadurch gekennzeichnet, dass** das Durchführen der Grauwertanpassung zwischen Ist-Röntgenbild (84) und Soll-Röntgenbild (86) den Schritt aufweist, in einem iterativen numerischen Optimierungsverfahren eine Anpassungsfunktion zum Abgleich von Grauwerthäufigkeitskurven von Ist-Röntgenbild (84) und Soll-Röntgenbild (86) anzuwenden.

5. Verfahren nach Anspruch 3 oder 4, **dadurch gekennzeichnet, dass** vor dem Durchführen der Grauwertanpassung zwischen Ist-Röntgenbild (84) und Soll-Röntgenbild (86) die Auflösung desjenigen mit der geringeren Auflösung auf die höhere Auflösung des anderen angepasst wird.

6. Verfahren nach einem der Ansprüche 2 bis 5, **dadurch gekennzeichnet, dass** beim Festlegen der relevanten zweidimensionalen Variationsgebiete (92) ausschließlich Gebiete berücksichtigt werden, die aus einer vorbestimmten Mindestzahl zusammenhängender Pixel (90) bestehen, die nach dem Subtrahieren eine Grauwertabweichung oberhalb eines vorbestimmten Schwellwerts zeigen.

7. Verfahren nach einem der Ansprüche 2 bis 6, **dadurch gekennzeichnet, dass** über die Pixel (90) jedes relevanten zweidimensionalen Variationsgebiets (92) eine Grauwertmittelung durchgeführt wird.

8. Verfahren nach einem der vorangehenden Ansprüche, **dadurch gekennzeichnet, dass** jedes relevante zweidimensionale Variationsgebiet (92) in den Ist-Röntgenbildern (84) oder den Soll-Röntgenbildern (86) graphisch hervorgehoben wird.

9. Verfahren nach einem der vorangehenden Ansprüche, **dadurch gekennzeichnet, dass** vor dem Rückprojizieren der relevanten zweidimensionalen Variationsgebiete (92) in den dreidimensionalen Computertomographiedatensatz (32) die zweidimensionalen Variationsgebiete (92) zu Rechtecken mit der mittleren Breite und mit der mittleren Länge des jeweiligen zweidimensionalen Variationsgebietes (92) vereinfacht werden.

10. Verfahren nach einem der vorangehenden Ansprüche, **dadurch gekennzeichnet, dass** beim Berechnen der mittleren Elektronendichte ($\rho$) des dreidimensionalen Variationskörpers (102) Gleichungssysteme für jede der zwei Projektionsrichtungen basierend auf dem Röntgenabschwächungszusammenhang

$$G(r) = e^{-\alpha \cdot \rho \cdot r}$$

numerisch gelöst werden, wobei G der numerische Grauwert und p die Elektronendichte ist.

**11.** Verfahren nach einem der vorangehenden Ansprüche, **dadurch gekennzeichnet, dass** das Berechnen von zu erwartenden geänderten Reichweiten der identifizierten Einzelstrahlen (112) des geplanten Bestrahlungsfeldes die Schritte aufweist, den differentiellen Energieverlust entlang des Eindringpfades des Einzelstrahls (112) zu bestimmen und die Reichweite des Eindringpfades des Einzelstrahls (112) durch Integration über den energieabhängigen differentiellen Energieverlust zu bestimmen.

**12.** Verfahren nach einem der vorangehenden Ansprüche, **dadurch gekennzeichnet, dass** das Berechnen von zu erwartenden geänderten Reichweiten der identifizierten Einzelstrahlen (112) des geplanten Bestrahlungsfeldes nur auf diejenigen Einzelstrahlen (112) beschränkt wird, welche die höchsten ursprünglichen Reichweiten betreffen.

**13.** Verfahren nach einem der vorangehenden Ansprüche, **dadurch gekennzeichnet, dass** das Darstellen von Informationen (114, 116, 118) über die berechneten geänderten Reichweiten den Schritt umfasst, die Informationen visuell in den Ist-Röntgenbildern (84) oder Soll-Röntgenbildern (86) als Angaben (114) über die Ausdehnung der Reichweitenveränderung darzustellen.

**14.** Verfahren nach einem der vorangehenden Absprüche, **dadurch gekennzeichnet, dass** das Darstellen von Informationen (114, 116, 118) über die berechneten geänderten Reichweiten den Schritt umfasst, in den Ist-Röntgenbildern (84) oder Soll-Röntgenbildern (86) eine Reichweitenvariation im distalen Endbereich des Bestrahlungsfeldes farblich zu kennzeichnen (116).

**15.** Verfahren nach einem der vorangehenden Ansprüche, **dadurch gekennzeichnet, dass** das Darstellen von Informationen (114, 116, 118) über die berechneten geänderten Reichweiten den Schritt umfasst, eine geänderte Dosisverteilung zu berechnen, die aus den berechneten geänderten Reichweiten folgt, und in den beiden Ist-Röntgenbildern (84) oder Soll-Röntgenbildern (86) Projektionen von Isodosisflächen der geänderten Dosisverteilung darzustellen.

**Claims**

**1.** Method for identification of possible changes in range of a planned radiation field before irradiating a patient (74) with charged particles, having the following steps:

carrying out computer tomography over at least a part of the patient (74) to create a three-dimensional computer tomography data set (32);

providing an X-ray system from two high-resolution X-ray detectors (80) which include an angle of 60° to 120°, preferably 75° to 105°, with one another, and two X-ray sources (78) which are opposite the X-ray detectors (80), wherein each one pair of X-ray source (78) and X-ray detector (80) defines one projection direction;

producing two actual X-ray images (84) of at least one part of the patient (74) in the two projection directions with the two X-ray detectors (80);

calculating two digitally reconstructed ideal X-ray images (86) from the computer tomography data set (32) corresponding to the two projection directions of the X-ray system;

undertaking a differential analysis to determine differences between the actual X-ray image (84) and the associated ideal X-ray image (86) for the two projection directions;

examining the relevance of the established differences with regard to spatial expansion and with regard to localisation in relation to the planned radiation field and determining relevant two-dimensional variation fields (92);

establishing a three-dimensional variation body (102) through back projection of the two-dimensional variation fields (92) into the three-dimensional computer tomography data set (32);

calculating a mean electron density (p) of the three-dimensional variation body (102) on the basis of the differences detected between the actual X-ray image (84) and the associated ideal X-ray image (86) for the two projection directions;

identifying individual rays (112) of the planned radiation field which pass through the three-dimensional variation body (102);

calculating the modified ranges to be expected of the identified individual rays (112) of the planned radiation field taking into account the modified electron density value (p) in the three-dimensional variation body (102); and

Illustrating or forwarding information (114, 116, 118) about the calculated modified ranges.

**2.** Method according to claim 1 **characterised in that** the step of carrying out a differential analysis for determining differences between the actual X-ray image (84) and the associated ideal X-ray image (86) comprises the step of subtracting numerical greyscale values from corresponding pixels (90) of the actual X-ray image (84) and ideal X-ray image (86) in order to determine a pixel-type greyscale deviation.

3. Method according to claim 2 **characterised in that** before the step of pixel-type subtracting the numeral greyscale values a greyscale adaption is carried out between the actual X-ray images (84) and the ideal X-ray images (86).

4. Method according to claim 3 **characterised in that** carrying out the greyscale adaption between the actual X-ray image (84) and the ideal X-ray image (86) has the step of applying in an iterative numerical optimization method an adaption function for compensating grey scale frequency curves of actual X-ray image (84) and ideal X-ray image (86).

5. Method according to claim 3 or 4 **characterised in that** before carrying out the greyscale adaption between the actual X-ray image (84) and the ideal X-ray image (86) the resolution of the one with the lower resolution is adapted to the higher resolution of the other one.

6. Method according to one of claims 2 to 5 **characterised in that** when establishing the relevant two-dimensional variation fields (92) only those fields are considered which consist of a predetermined minimum number of associated pixels (90) which after the subtraction show a greyscale deviation above a predetermined threshold value.

7. Method according to one of claims 2 to 6 **characterised in that** a greyscale averaging is carried out on the pixels (90) of each relevant two-dimensional variation field (92).

8. Method according to one of the preceding claims **characterised in that** each relevant two-dimensional variation field (92) in the actual X-ray images (84) or the ideal X-ray images (86) is graphically emphasized.

9. Method according to one of the preceding claims **characterised in that** before projecting the relevant two-dimensional variation fields (92) back into the three-dimensional computer tomography data set (92) the two-dimensional variation fields (92) are simplified into rectangles with the average width and with the average length of the relevant two-dimensional variation field (92).

10. Method according to one of the preceding claims **characterised in that** when calculating the mean electron density (p) of the three-dimensional variation body (102) equation systems for each of the two projection directions are numerically solved based on the X-ray attenuation relationship

$$G(r) = e^{-\alpha.p.r}$$

wherein G is the numerical greyscale value and p the electron density.

11. Method according to one of the preceding claims **characterised in that** calculating anticipated changed ranges of the identified individual rays (112) of the planned radiation field has the steps determining the differential energy loss along the penetration path of the individual ray (112) and determining the range of the penetration path of the individual ray (112) through integration over the energy-dependent differential energy loss.

12. Method according to one of the preceding claims **characterised in that** calculating the anticipated changed ranges of the identified individual rays (112) of the planned radiation field is restricted only to those individual rays (112) which concern the highest original ranges.

13. Method according to one of the preceding claims **characterised in that** illustrating the information (114, 116, 118) on the calculated changed ranges comprises the step of showing the information visually in the actual X-ray images (84) or ideal X-ray images (86) as details about the extension of the range change.

14. Method according to one of the preceding claims **characterised in that** illustrating the information (114, 116, 118) on the calculated changed ranges comprises the step of marking in colour (116) in the actual X-ray images (84) or ideal X-ray images (86) a range variation in the distal end region of the radiation field.

15. Method according to one of the preceding claims **characterised in that** illustrating the information (114, 116, 118) on the calculated changed ranges comprises the step of calculating a changed dose distribution, which follows from the calculated changed ranges, and showing in the two actual X-ray images (84) or ideal X-ray images (86) projections of isodose areas of the changed dose distribution.

**Revendications**

1. Procédé pour l'identification de possibles modifications de la portée d'un champ de rayonnement planifié avant l'irradiation d'un patient (74) avec des particules chargées, comprenant les étapes suivantes :

mise en oeuvre d'une tomographie par ordinateur au-dessus d'une partie au moins du patient (74) pour produire un jeu de données tridimensionnelles (32) ;
fourniture d'un système de radiographie à partir de deux détecteurs de rayons à haute résolution (80) qui forment ensemble un angle de 60° à 120°, de préférence de 75° à 105°, et deux sources de rayons (78) opposées aux détecteurs de rayons (80), chaque paire de source de rayons (78) et détecteur de rayons (80) définissant respectivement une direction de projection ;
réalisation de deux radiographies réelles (84) d'une partie au moins du patient (74) dans les deux directions de projection avec les deux détecteurs de rayons (80) ;
calcul de deux radiographies théoriques (86) reconstruites numériques à partir du jeu de données de tomographie par ordinateur (32) conformément aux deux directions de projection du système de radiographie ;
mise en oeuvre d'une analyse différentielle pour constater des différences entre la radiographie réelle (84) et la radiographie théorique (86) associée pour les deux directions de projection ;
vérification de la pertinence des différences constatées en ce qui concerne l'extension spatiale et en ce qui concerne la localisation par rapport au champ de rayonnement planifié et la définition de domaines de variation bidimensionnels pertinents (92) ;
définition d'un corps de variation tridimensionnel (102) par rétroprojection des domaines de variation bidimensionnels (92) dans le jeu de données de tonographie par ordinateur (32) ;
calcul d'une densité électronique moyenne (p) du corps de variation tridimensionnel (102) sur la base des différences constatées entre la radiographie réelle (84) et la radiographie théorique (86) associée pour les deux directions de projection ;
identification de rayons individuels (112) du champ de rayonnement planifié qui traversent le corps de variation tridimensionnel (102) ;
calcul des portées modifiées escomptées de rayons individuels (112) identifiés du champ de rayonnement planifié avec prise en compte de la valeur de densité électronique (p) modifiée dans le corps de variation tridimensionnel (102) ; et
représentation ou transmission d'informations (114, 116, 118) sur les portées modifiées calculées.

2. Procédé selon la revendication 1, **caractérisé en ce que** l'étape de mise en oeuvre d'une analyse différentielle permettant de constater des différences entre la radiographie réelle (84) et la radiographie théorique (86) associée comprend l'étape de soustraction de valeurs de gris numériques de pixels (90) en correspondance de la radiographie réelle (84) et de la radiographie théorique (86), pour déterminer un écart de valeur de gris pixel par pixel.

3. Procédé selon la revendication 2, **caractérisé en ce que**, avant l'étape de soustraction pixel par pixel des valeurs de gris numériques, une adaptation de valeurs de gris entre les radiographies réelles (84) et les radiographies théoriques (86) est mise en oeuvre.

4. Procédé selon la revendication 3, **caractérisé en ce que** la mise en oeuvre de l'adaptation de valeur de gris entre la radiographie réelle (84) et la radiographie théorique (86) comprend l'étape d'application, au cours d'un procédé d'optimisation numérique itératif, d'une fonction d'adaptation permettant l'équilibrage de courbes de fréquence de valeurs de gris de la radiographie réelle (84) et de la radiographie théorique (86).

5. Procédé selon la revendication 3 ou 4, **caractérisé en ce que**, avant la mise en oeuvre de l'adaptation de valeurs de gris entre la radiographie réelle (84) et la radiographie théorique (86), la résolution de celle présentant la résolution la plus basse est adaptée à la résolution la plus haute de l'autre.

6. Procédé selon l'une quelconque des revendications 2 à 5, **caractérisé en ce que**, lors de la définition des domaines de variation bidimensionnels pertinents (92), seuls les domaines constitués d'un nombre minimal prédéfini de pixels (90) cohérents présentant après la soustraction un écart de valeurs de gris supérieur à une valeur seuil prédéfinie sont pris en compte.

7. Procédé selon l'une quelconque des revendications 2 à 6, **caractérisé en ce qu'**un calcul de la moyenne des valeurs de gris est mis en oeuvre à l'aide des pixels (90) de chaque domaine de variation bidimensionnel pertinent (92).

8. Procédé selon l'une quelconque des revendications précédentes, **caractérisé en ce que** chaque domaine de variation bidimensionnel pertinent (92) dans les radiographies réelles (84) ou les radiographies théoriques (86) est mis en évidence graphiquement.

9. Procédé selon l'une quelconque des revendications précédentes, **caractérisé en ce que**, avant la rétroprojection des domaines de variation bidimensionnels pertinents (92) dans le jeu de données de tomographie par ordinateur tridimensionnelles (32), les domaines de variation bidimensionnels (92) sont réduits en rectangles présentant la largeur moyenne et la longueur moyenne du domaine de variation bidimensionnel (92) respectif.

10. Procédé selon l'une quelconque des revendications précédentes, **caractérisé en ce que**, lors du calcul de la densité électronique moyenne (p) du corps de variation tridimensionnel (102), des systèmes d'équation pour chacune des deux directions de projection sont résolus numériquement sur la base de la relation d'atténuation des rayons

$$G(r) = e^{-\alpha p r}$$

G étant la valeur de gris numérique et p étant la densité électronique.

11. Procédé selon l'une quelconque des revendications précédentes, **caractérisé en ce que** le calcul de portées modifiées escomptées des rayons individuels (112) identifiés du champ de rayonnement planifié comprend les étapes de détermination de la perte d'énergie différentielle le long du trajet de pénétration du rayon individuel (112) et de détermination de la portée du trajet de pénétration du rayon individuel (112) par intégration au moyen de la perte d'énergie différentielle dépendant de l'énergie.

12. Procédé selon l'une quelconque des revendications précédentes, **caractérisé en ce que** le calcul des portées modifiées escomptées des rayons individuels (112) identifiés du champ de rayonnement planifié n'est limité qu'aux rayons individuels (112) qui concernent les portées initiales les plus élevées.

13. Procédé selon l'une quelconque des revendications précédentes, **caractérisé en ce que** la représentation d'informations (114, 116, 118) concernant les portées modifiées calculées comprend l'étape de représentation visuelle des informations dans les radiographies réelles (84) ou les radiographies théoriques (86) comme indications (114) de l'étendue de la modification de la portée.

14. Procédé selon l'une quelconque des revendications précédentes, **caractérisé en ce que** la représentation des informations (114, 116, 118) concernant les portées modifiées calculées comprend l'étape de marquage (116) en couleur dans les radiographies réelles (84) ou les radiographies théoriques (86) d'une variation de la portée dans la zone d'extrémité distale du champ de rayonnement.

15. Procédé selon l'une quelconque des revendications précédentes, **caractérisé en ce que** la représentation d'informations (114, 116, 118) concernant les portées modifiées calculées comprend l'étape de calcul d'une distribution de dose modifiée découlant des portées modifiées calculées, et de représentation dans les deux radiographies réelles (84) ou radiographies théoriques (86) des projections de surfaces isodosiques de la distribution de dose modifiée.

Fig. 1

Fig. 2

Fig. 3

Fig. 4(a)

Fig. 4(b)

Fig. 5

12

14    18    20    22

16

Fig. 6

Fig. 7

Fig. 8

84

84

80

80

88

74

Fig. 9(a)

86

86

74

Fig. 9(b)

87

87

Fig. 9(c)

Häufigkeit

Soll-Röntgenbild

Grauwert $G_{soll}$

Häufigkeit

Fig. 10(a)

Ist-Röntgenbild

Grauwert $G_{ist}$

$G_{ist}$

100%

Fig. 10(b)

$G_{ist}{}^* = F(G_{ist})$

$G_{ist}{}^*$

100%

Fig. 10(c)

Häufigkeit

—— Soll-Röntgenbild

-- Ist-Röntgenbild, angepasst

$G_{soll}$

| 5 | 6 | 5 | 4 | 3 | 6 |
|---|---|---|---|---|---|
| 4 | 3 | 6 | 5 | 4 | 5 |
| 5 | 6 | 5 | 4 | 6 | 3 |
| 5 | 7 | 4 | 3 | 5 | 6 |
| 6 | 5 | 5 | 4 | 6 | 5 |
|   |   |   |   |   |   |

## Fig. 11(a)

Soll-Röntgenbild

| 5 | 5 | 4 | 6 | 4 | 5 |
|---|---|---|---|---|---|
| 4 | 5 | 6 | 5 | 4 | 6 |
| 5 | 29 | 35 | 37 | 33 | 6 |
| 6 | 30 | 32 | 34 | 35 | 33 |
| 7 | 35 | 33 | 36 | 6 | 5 |
|   |   |   |   |   |   |

## Fig. 11(b)

Ist-Röntgenbild

| 0 | -1 | -1 | 2 | 1 | -1 |
|---|---|---|---|---|---|
| 0 | 2 | 0 | 0 | 0 | 1 |
| 0 | 23 | 30 | 33 | 27 | 3 |
| 1 | 23 | 28 | 31 | 30 | 27 |
| 1 | 30 | 28 | 32 | 0 | 0 |
|   |   |   |   |   |   |

## Fig. 11(c)

Differenzbild
    Ist-Röntgenbild
    minus Soll-Röntgenbild

90

Fig. 12(a)

92

Fig. 12(b)

Fig. 13(a)

Fig. 13(b)

Fig. 13(c)

| | I | II | III | IV |
|---|---|---|---|---|
| linkes Differenzbild | X | X | O | O |
| rechtes Differenzbild | X | O | O | X |

Fig. 14

Fig. 15

Fig. 16

Fig. 17(a)

Fig. 17(b)

Fig. 17(c)